# EUROPÄISCHE PATENTSCHRIFT

(11) **EP 1 678 190 B1**
(45) Veröffentlichungstag und Bekanntmachung des Hinweises auf die Patenterteilung: **21.11.2012**
(21) Anmeldenummer: 04790697.9
(22) Anmeldetag: 21.10.2004
(51) Int. Cl.: C07D 213/26, C07D 213/30, C07F 9/58, C07F 15/00, H01L 51/00

(54) **METALLKOMPLEXE MIT BIPODALEN LIGANDEN**
METAL COMPLEXES WITH BIPODAL LIGANDS
COMPLEXES METALLIQUES A LIGANDS BIPODES

(30) Priorität: 30.10.2003 DE 10350722
(43) Veröffentlichungstag der Anmeldung: 12.07.2006
(73) Patentinhaber: Merck Patent GmbH, 64293 Darmstadt (DE)
(72) Erfinder: STÖSSEL, Philipp, 60487 Frankfurt am Main (DE); GERHARD, Anja, 97209 Veitshöchheim (DE)
(86) Internationale Anmeldenummer: PCT/EP2004/011890
(87) Internationale Veröffentlichungsnummer: WO 2005/042550

(56) Entgegenhaltungen:
- WO-A-01/70395
- WO-A-02/068435
- WO-A1-2004/108875
- US-B1- 6 613 583
- SLUGOVC C ET AL: "Generation of Heteroatom-Substituted Carbene Complexes of Iridium by Double C-H Activation of Ether and Amine Substrates" ANGEWANDTE CHEMIE. INTERNATIONAL EDITION, VERLAG CHEMIE. WEINHEIM, DE, Bd. 39, Nr. 12, 2000, Seiten 2158-2160, XP002284927 ISSN: 0570-0833
- DATABASE CA [Online] CHEMICAL ABSTRACTS SERVICE, COLUMBUS, OHIO, US; CONSTABLE, EDWIN C. ET AL: "A double helix dinickel(II) complex with a 1,3-phenylene spacer group through spontaneous self organization" XP002312313 gefunden im STN Database accession no. 1992:186499 & ANGEWANDTE CHEMIE , 104(2), 218-20 (SEE ALSO ANGEW. CHEM., INT. ED. ENGL., 1992, 31(2), 230-2) CODEN: ANCEAD; ISSN: 0044-8249, 1992,
- BODAR-HOUILLON F ET AL: "Synthesis and Luminescence Properties of a New Tripode Containing 2,2@?-Bipyrazine Subunits: The tris-[6-methyl-2,2@?-bipyrazine-2-yl)m ethyl]amine" TETRAHEDRON LETTERS, ELSEVIER SCIENCE PUBLISHERS, AMSTERDAM, NL, Bd. 36, Nr. 6, 6. Februar 1995 (1995-02-06), Seiten 865-868, XP004028691 ISSN: 0040-4039
- PALILIS L C ET AL: "HIGH EFFICIENCY MOLECULAR ORGANIC LIGHT-EMITTING DIODES BASED ON SILOLE DERIVATIVES AND THEIR EXCIPLEXES" ORGANIC ELECTRONICS, ELSEVIER, AMSTERDAM, NL, Bd. 4, Nr. 2/3, September 2003 (2003-09), Seiten 113-121, XP001177136 ISSN: 1566-1199
- DATABASE BEILSTEIN [Online] BEILSTEIN INSTITUTE FOR ORGANIC CHEMISTRY, FRANKFURT-MAIN, DE; 1997, XP002312314 & HO, PAUL KWOK-KEUNG ET AL.: J.CHEM.SOC. DALTON TRANS., Nr. 9, 1996, Seiten 1829-1834,
- DATABASE BEILSTEIN [Online] BEILSTEIN INSTITUTE FOR ORGANIC CHEMISTRY, FRANKFURT-MAIN, DE; 1993, XP002312315 Database accession no. BRN 5890400; BRN 5896018 & LHOTAK, P. ET AL.: COLLECT. CZECH. COMMUN., Bd. 57, Nr. 9, 1992, Seiten 1937-1946,
- DATABASE BEILSTEIN [Online] BEILSTEIN INSTITUTE FOR ORGANIC CHEMISTRY, FRANKFURT-MAIN, DE; 1991, XP002312316 Database accession no. BRN 4622990 & MATAKA ET AL.: J. HETEROCYCL. CHEM., Nr. 19, 1982, Seiten 1481-1488,
- PIGUET C ET AL: "Tridentate binding units as structural patterns for the design of nine-coordinate lanthanide building blocks with predetermined properties", JOURNAL OF ALLOYS AND COMPOUNDS, ELSEVIER SEQUOIA, LAUSANNE, CH, vol. 303-304, 1 May 2000 (2000-05-01), pages 94-103, XP004204340, ISSN: 0925-8388, DOI: 10.1016/S0925-8388(00)00656-3
- ZHANG H. ET AL: "Synthesis of Binucleating Ligands of Pyridylphenol", SYNTHETIC COMMUNICATIONS, vol. 31, no. 8, 2001, pages 1129-1139, XP009120142,
- BÖNNEMANN H.; BRINKMANN R.: "Eine kobalt-katalysierte Einstufen-Synthese von Dipyridinen", SYNTHESIS, no. 9, 1975, pages 600-603,
- HANNON M.J.; MAYERS P.C.; TAYLOR P.C.: "Preparation of substituted tris(2-pyridyl)methanol derivatives as mimics of the metal binding site of carbonic anhydrase", TETRAHEDRON LETTERS, vol. 39, no. 46, 1998, pages 8509-8512, XP004139456,

## Beschreibung

Chelatkomplexe und Organometallverbindungen werden in naher Zukunft als funktionelle Materialien in einer Reihe verschiedenartiger Anwendungen, die im weitesten Sinne der Elektronikindustrie zugerechnet werden können, Einsatz finden. Bei den auf organischen Komponenten basierenden organischen Elektrolumineszenz-Vorrichtungen (allg. Beschreibung des Aufbaus vgl. US 4,539,507 und US 5,151,629) bzw. deren Einzelbauteilen, den organischen lichtemittierenden Dioden (OLEDs), ist die Markteinführung bereits erfolgt, wie die Autoradios bzw. Digitalkameras mit "Organischem Display" der Firmen Pioneer und Kodak belegen. Weitere derartige Produkte stehen kurz vor der Einführung, Trotz allem sind hier noch deutliche Verbesserungen nötig, um diese Displays zu einer echten Konkurrenz zu den derzeit marktbeherrschenden Flüssigkristallanzeigen (LCD) zu machen.

Eine Entwicklung hierzu ist die Verbesserung von Elektronentransportmaterialien und blauen Singulettemittern auf Basis von Metall-Chelatkomplexen, wobei hier insbesondere Aluminium- und Lanthan-Chelat-Komplexe von Interesse sind. Eine weitere Entwicklung, die sich in den letzten Jahren abzeichnet, ist der Einsatz metallorganischer Komplexe, die Phosphoreszenz statt Fluoreszenz zeigen (M. A. Baldo, S. Lamansky, P. E. Burrows, M. E. Thompson, S. R. Forrest, Appl. Phys. Lett., 1999, 75, 4-6). Aus theoretischen Spin-statistischen Gründen ist unter Verwendung metallorganischer Verbindungen als Phosphoreszenzemitter eine bis zu vierfache Energie- und Leistungseffizienz möglich. Ob sich diese Entwicklung durchsetzen wird, hängt davon ab, ob entsprechende Device-Kompositionen gefunden werden können, die diese Vorteile (Triplett-Emission = Phosphoreszenz gegenüber Singulett-Emission = Fluoreszenz) auch in den OLEDs umsetzen können. Als wesentliche Bedingungen für praktische Anwendung sind hier insbesondere eine hohe operative Lebensdauer, eine hohe Stabilität gegenüber Temperaturbelastung und eine niedrige Einsatz- und Betriebsspannung, um mobile Applikationen zu ermöglichen, zu nennen.

In beiden Fällen muß der effiziente chemische Zugang zu den entsprechenden Chelat-Komplexen bzw. Organometall-Verbindungen gegeben sein. Von besonderem Interesse ist dies jedoch vor dem Hintergrund der Knappheit der Edelmetalle Ruthenium, Osmium, Rhodium, Iridium, Palladium, Platin und Gold.

In der Literatur wurden bis jetzt zwei grundlegende Bautypen von OLEDs, die Fluoreszenz- bzw. Phosphoreszenzemitter als farbgebende Komponenten enthalten, beschrieben, die sich in ihrem Schichtaufbau unterscheiden. Diese OLED-Typen werden z. B. in WO 04/058911 ausführlich beschrieben.

Die Kenndaten der OLEDs gemäß dem Stand der Technik zeigen unter anderem folgende Schwachpunkte:
1. Die operative Lebensdauer ist in den meisten Fällen noch deutlich zu gering, was einer Einführung von langlebigen OLEDs im Markt entgegensteht.
2. Aus den Effizienz-Helligkeits-Kurven geht hervor, daß die Effizienz mit steigender Helligkeit häufig stark abnimmt. Dies bedeutet, daß die in der Praxis notwendigen großen Helligkeiten nur über eine hohe Leistungsaufnahme erreicht werden können. Große Leistungsaufnahmen setzen aber große Batterieleistungen portabler Geräte (Mobile-Phones, Laptops, etc.) voraus. Außerdem kann die großen Leistungsaufnahme, die zum großen Teil in Wärme umgesetzt wird, zur thermischen Schädigung des Displays führen.

Bei der oben erläuterten OLED-Vorrichtung wurden bzw. werden die oben genannten Funktionsmaterialien intensiv optimiert.

Seit einiger Zeit werden Metallkomplexe als ETM (Elektronentransportmaterial) (z. B. AIQ₃, C. W. Tang et al., Applied Phys. Lett., 1987, 51(12), 913; ZnQ₂ S.-J. Jung et al., J. Korean Electrochemical Society, 2000, 3(1), 1), als HBM (Lochblockiermaterial) (z. B. B-AIQ₃, R. Kwong et al., Applied Physics Letters, 2002, 81(1), 162), als Matrixmaterial in der EML (Emissionsschicht) (z. B. B-AIQ₃, C. H. Chen et al., Proceedings of SPIE-The International Society for Optical Engineering, 1998, 3421, 78), als Singulett-Emitter (z. B. AlQ₃, ZnQ₂ und andere Komplexe, S. Tokito et al., Synthetic Metals, 2000, 111 - 112, 393) und als Triplett-Emitter (z. B. Ir(PPy)₃, WO 00/70655; z. B. Ir(TPy)₃ und Ir(BTPy)₃, S. Okada et al., Proceedings of the SID, 2002, 52.2, 1360) eingesetzt. Triplettemitter auf der Basis von Platinkomplexen sind ebenfalls seit einiger Zeit bekannt, wobei neben Komplexen zweizähniger Liganden (z. B. Brooks et al., Inorg. Chem., 2002, 41, 3055-3066) auch solche vierzähniger makrocyclischer Liganden (z. B. PtOEP, L. R. Milgrom, Polyhedron, 1988, 7(1), 57; M. A. Baldo, Nature, 1998, 395(6698), 151-154) bekannt sind. Diese Komplexe des zweiwertigen Platins (d⁸-Konfiguration) sind, wie die überwiegende Zahl der Platin(II)-Komplexe, planar bzw. fast planar gebaut. Im Feststoff aggregieren diese planaren Komplexeinheiten derart, daß es zur Ausbildung starker und häufig kooperativer Ligand-Ligand-, Metall-Metall- oder Ligand-Metall-Wechselwirkungen kommt. Neben den individuellen, für jedes Material spezifischen Schwachpunkten besitzt die Klasse der bekannten Metallkomplexe generelle Schwachpunkte, die im folgenden kurz aufgezeigt werden:
1. Viele der bekannten Metallkomplexe, insbesondere solche, die Hauptgruppenmetalle wie Aluminium oder Übergangsmetalle mit d¹⁰-Konfiguration wie Zink enthalten, weisen eine zum Teil erhebliche Hydrolyseempfindlichkeit auf, die so weit gehen kann, daß der Metallkomplex schon nach kurzer Exposition an Luft merklich zersetzt wird. Andere dagegen, wie zum Beispiel das als Elektronentransportmaterial verwendete AIQₛ und ZnQ₂, neigen zur Anlagerung von Wasser. Die starke Hygroskopie dieser und ähnlicher Aluminium- und Zinkkomplexe ist ein entscheidender praktischer Nachteil. AIQ₃, welches unter normalen Bedingungen synthetisiert und aufbewahrt wird, enthält neben den Hydroxychinolin-Liganden immer noch ein Molekül Wasser pro Komplex-Molekül (vgl. z. B.: H. Schmidbaur et al., Z. Naturforsch., 1991, 46b, 901-911). Dieses ist extrem schwer zu entfernen. Für die Verwendung in OLEDs müssen AIQ₃ und ZnQ₂ deshalb in komplizierten, mehrstufigen Sublimationsverfahren aufwendig gereinigt und im Anschluß daran unter Wasserausschluß in einer Schutzgasatmosphäre gelagert und gehandhabt werden. Weiterhin wurden große Schwankungen in der Qualität einzelner AIQ₃-Chargen, sowie eine schlechte Lagerstabilität festgestellt (S. Karg, E-MRS Konferenz, **2000**, Straßburg).
2. Viele der bekannten Metallkomplexe besitzen eine geringe thermische Stabilität. Diese führt bei einer Vakuumdeposition zwangsläufig immer zur Freisetzung organischer Pyrolyseprodukte, die zum Teil schon in geringen Mengen die operative Lebensdauer der OLEDs erheblich verringern (z. B.: R. G. Charles, J. Inorg. Nucl. Chem., 1963, 25, 45; über die thermische Stabilität von MQ₂).
3. Ebenso bedingt die starke Wechselwirkung der Komplexeinheiten im Feststoff, insbesondere bei planaren Komplexen von d⁸-Metallen wie Platin(II), die Aggregation der Komplexeinheiten in der Emitterschicht, sofern der Dotierungsgrad etwa 0.1 % überschreitet, was nach derzeitigem Stand der Technik der Fall ist. Diese Aggregation führt bei Anregung (optisch oder elektrisch) zur Bildung sogenannter Excimere bzw. Exciplexe. Diese Aggregate weisen häufig eine unstrukturierte breite Emissionsbande auf, was die Erzeugung von reinen Grundfarben (RGB) erheblich erschwert bzw. vollständig unmöglich macht. In der Regel sinkt auch die Effizienz für diesen Übergang.
3. Aus dem oben gesagten geht außerdem hervor, daß die Emissionsfarbe stark vom Dotierungsgrad abhängt, einem Parameter, der insbesondere in großen Produktionsanlagen nur mit erheblichem technischen Aufwand exakt kontrolliert werden kann.

Es bestand daher ein Bedarf an alternativen Verbindungen, die die oben genannten Schwachpunkte nicht aufweisen.

Es wurde überraschend gefunden, daß Metallkomplexe vierzähniger chelatisierender, nicht makrocyclischer Liganden hervorragende Eigenschaften bei der Verwendung als Elektronentransportmaterial, als Lochblockiermaterial, als Matrixmaterial in der EL, als Singulett-Emitter oder auch als Triplett-Emitter zeigen, wobei die jeweilige, konkrete Funktion durch die geeignete Wahl des Metalls und des geeigneten zugehörigen Liganden bestimmt wird. Diese Klasse der Metallkomplexe und deren Verwendung als Funktionsmaterialien in opto-elektronischen Komponenten ist neu und bisher in der Literatur nicht beschrieben, ihre effiziente Darstellung und Verfügbarkeit als Reinstoff ist hierfür aber von großer Bedeutung.

Aus WO 2002/068435 sind Rhodium- und Iridiumkomplexe mit Phenylpyridinliganden bekannt, welche am Phenylring mit Halogenatomen substituiert sind. Komplexe, in denen zwei Liganden durch eine verbrückende Einheit miteinander verbunden sind, sind nicht offenbart.

Aus WO 2004/108857 sind Metallkomplexe mit tridentatem bzw. tetradentatem Liganden bekannt. Dabei werden Komplexe offenbart, bei denen zwei bidentate Teilliganden vom Typ Phenylpyridin über eine Carbonylgruppe oder eine Alkylengruppe miteinander verbunden sind.

C. Piguet et al. (Journal of Alloys and Coumpounds, 2000, 303-304, S. 94-103) offenbaren Zink- und Lanthankomplexe mit tetradentatem Liganden. Dabei koordiniert jeder tetradentate Ligand an zwei Metallatome. Eine Anwendung dieser Komplexe in organischen Elektrolumineszenzvorrichtungen ist nicht offenbart.

Gegenstand der vorliegenden Erfindung sind somit elektisch neutrale Verbindungen der Struktur 1, 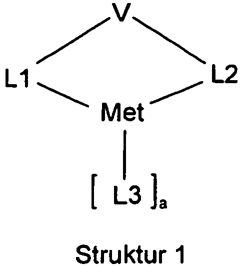 dadurch gekennzeichnet, daß sie ein Metall Met enthalten, koordiniert an einen vierzähnig chelatisierenden Liganden Lig gemäß Struktur 2, 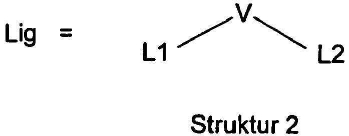 wobei V eine verbrückende Einheit ist, dadurch gekennzeichnet, daß sie 1 bis 40 Atome aus der vierten, fünften und/oder sechsten Hauptgruppe enthält und die zwei Teilliganden L1 und L2 , die gleich oder verschieden bei jedem Auftreten sein können, kovalent miteinander verbindet, und wobei die zwei Teilliganden L1 und L2 der Struktur 3 genügen, 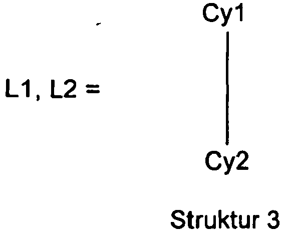 wobei Cy1 und Cy2, gleich oder verschieden bei jedem Auftreten, je einem substituierten oder unsubstituierten, gesättigten, ungesättigten oder aromatischen Homo- oder Heterocyclus entsprechen, bevorzugt einem aromatische Cyclus, der jeweils über ein Ringatom oder über ein exocyclisch an den Homo- oder Heterocyclus gebundenes Atom ionisch, kovalent oder koordinativ an das Metall (Met) gebunden ist;
und wobei L3 gleich oder verschieden bei jedem Auftreten ein ein- oder zweizähniger, neutraler oder monoanionischer Ligand ist und wobei a gleich 0, 1 oder 2 ist;
dabei sind die folgenden Verbindungen von der Erfindung ausgenommen: 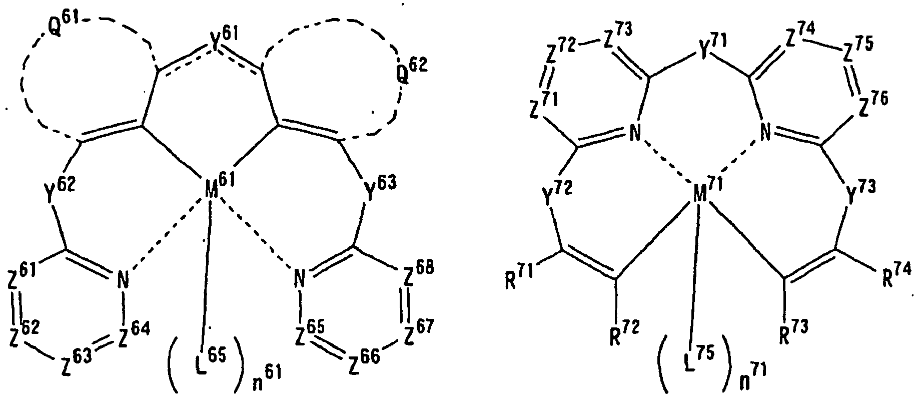 worin gilt:
- M⁶¹, M⁷¹: ist ein Metallion;
- Y⁶¹, Y⁷¹: ist eine Carbonylgruppe oder eine Alkylengruppe;
- Y⁶², Y⁶³, Y⁷², Y⁷³: ist jeweils eine Einfachbindung;
- Q⁶¹, Q⁶²: stellen eine Gruppe dar, die einen Ring bilden;
- Z⁶¹ bis Z⁶⁸, Z⁷¹ bis Z⁷⁶: stellen jeweils ein substituiertes oder unsubstituiertes Kohlenstoffatom oder ein Stickstoffatom dar;
- R⁷¹ bis R⁷⁴: ist jeweils ein Substituent, wobei R⁷¹ und R⁷² aneinander binden und so einen Ring bilden und wobei R⁷³ und R⁷⁴ aneinander binden und so einen Ring bilden;
- L⁶⁵, L⁷⁵: ist ein Ligand, der an das Metall koordiniert;
- n⁶¹, n⁷¹: ist 0 bis 4.

Die Brücke V ist dadurch gekennzeichnet, daß sie die Bildung von einkernigen Metallkomplexen der Struktur 1 fördert und die Bildung von Koordinationspolymeren bei Umsetzung des Liganden der Struktur 2 mit Metallverbindungen nicht oder nur in untergeordnetem Maße auftritt.

Die Homo- oder Heterocyclen Cy1 und Cy2 können auch zusätzlich über Substituenten miteinander verknüpft sein und so ein polycyclisches, aliphatisches oder aromatisches Ringsystem aufspannen. Ebenso können sie über eine gemeinsame Kante statt über eine Einfachbindung miteinander verknüpft sein.

Die efindungsgemäßen Verbindungen der Struktur 1 sind elektrisch neutral.

Bevorzugt sind erfindungsgemäße Verbindungen der Struktur 1, dadurch gekennzeichnet, daß L1 = L2 ist.

Weiterhin bevorzugt sind erfindungsgemäße Verbindungen gemäß Struktur 1, dadurch gekennzeichnet, dass Cy1 ungleich Cy2 ist. Dabei bindet bevorzugt einer der beiden Cyclen über eine Metall-Kohlenstoff-Bindung und der andere über ein Donoratom ungleich Kohlenstoff, besonders bevorzugt über N, P oder S.

Bevorzugt sind erfindungsgemäße Verbindungen gemäß Struktur 1, dadurch gekennzeichnet, daß die verbrückende Einheit V 1 bis 40 Atome aus der vierten, fünften und/oder sechsten Hauptgruppe (Gruppe 13, 14, 15 oder 16 gemäß IUPAC) oder einen 3- bis 6-gliedrigen Homo- oder Heterocyclus aufweist. Diese bilden das Grundgerüst der verbrückenden Einheit. Besonders bevorzugt sind Verbindungen der Struktur 1, dadurch gekennzeichnet, daß die Verknüpfungseinheit V 1 bis 6 verbrückende Atome enthält oder ein 3- bis 6-gliedriger Homo- oder Heterocyclus ist. Dabei kann die verbrückende Einheit V auch unsymmetrisch aufgebaut sein, d. h. die Verknüpfung von V zu L1 und L2 muß nicht identisch sein.

Besonders bevorzugt sind Verknüpfungseinheiten V, bei denen gilt:
- V: ist C=O, C=NR¹, C=S, CR₂, CR(OH), CR(OR¹), C(NR¹)₂, -(CR₂)R₂C(CR₂)-, -(CR₂CR₂)R₂C(CR₂CR₂)-, -(SiR₂)R₂C(SiR₂)-, -(SiR₂CR₂)R₂C(CR₂SiR₂)-, - (CR₂SiR₂)R₂C(SiR₂CR₂)-, -(SiR₂SiR₂)R₂C(SiR₂SiR₂)-, cis-RC=CR, 1,2-C₆H₄, 1,3-C₆H₄, SiR₂, Si(OH)₂, Si(OR¹)₂, -(CR₂)R₂Si(CR₂)-, -(CR₂CR₂)R₂Si(CR₂CR₂)-, -(SiR₂)R₂Si(SiR₂)-, -(SiR₂CR₂)R₂Si(CR₂SiR₂)-, -(CR₂SiR₂)R₂Si(SiR₂CR₂)-, -(SiR₂SiR₂)R₂Si(SiR₂SiR₂)-, R¹N, -(CR₂)R¹N(CR₂)-, -(CR₂CR₂)R¹N(CR₂CR₂)-, FP, FPO, R¹P, R¹As, R¹Sb, R¹Si, R¹PO, R¹AsO, R¹SbO, R¹BiO, R¹PSe, R¹AsSe, R¹SbSe, R¹BiSe, R¹PTe, R¹AsTe, R¹SbTe, R¹SiTe, -O-R¹PO-O-, -O-(R¹O)PO-O-, -CR₂O-R¹PO-OCR₂-, -OCR₂-R¹PO-CR₂O-, O, S, Se, -(CR₂)O(CR₂)-, -(CR₂)S(CR₂)-, -(CR₂)(O)S(CR₂)- oder -(CR₂)(O)₂S(CR₂)- oder entsprechende unsymmetrische Analoga;
- R: ist gleich oder verschieden bei jedem Auftreten H, F, Cl, Br, I, NO₂, CN, eine geradkettige, verzweigte oder cyclische Alkyl- oder Alkoxygruppe mit 1 bis 20 C-Atomen, wobei eine oder mehrere nicht benachbarte CH₂-Gruppen durch -R¹C=CR¹-, -C≡C-, Si(R¹)₂, Ge(R¹)₂, Sn(R¹)₂, C=O, C=S, C=Se, C=NR¹, -O-, -S-, -NR¹- oder -CONR¹- ersetzt sein können und wobei ein oder mehrere H-Atome durch F ersetzt sein können, oder eine Aryl-, Aryloxy- oder Heteroarylgruppe mit 1 bis 14 C-Atomen, die durch einen oder mehrere nicht aromatische Reste R substituiert sein kann, wobei mehrere Substituenten R wiederum ein weiteres mono- oder polycyclisches, aliphatisches oder aromatisches Ringsystem aufspannen können; und
- R¹, R²: ist gleich oder verschieden bei jedem Auftreten H oder ein aliphatischer oder aromatischer Kohlenwasserstoffrest mit 1 bis 20 C-Atomen.

Insbesondere bevorzugt sind Metallkomplexe gemäß den Verbindungen (1) bis (8) gemäß Schema 1, die jeweils noch einen oder zwei zusätzliche Liganden L3 tragen können, wie oben beschrieben. 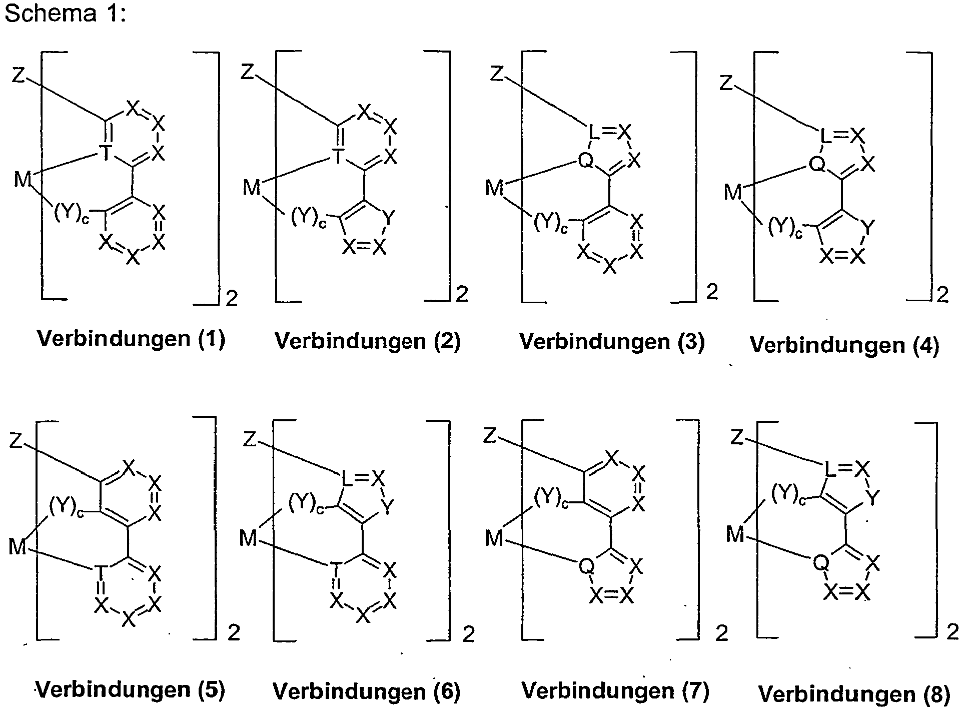 wobei R, R¹ und R² dieselbe Bedeutung haben, wie oben beschrieben, und die weiteren Symbole und Indizes folgende Bedeutung haben:
- M: ist Be, Mg, Ca, Sr, Ba, Al, Ga, In, Tl, Sc, Y, La, Cr, Mo, W, Fe, Ru, Os, Co, Rh, Ir, Ni, Pd, Pt, Cu, Ag, Au, Zn, Cd oder Hg;
- L: ist gleich oder verschieden bei jedem Auftreten C, N oder P;
- Q: ist gleich oder verschieden bei jedem Auftreten N, O, S, Se oder Te;
- T: ist gleich oder verschieden bei jedem Auftreten N oder P;
- X: ist gleich oder verschieden bei jedem Auftreten CR, N oder P;
- Y: ist gleich oder verschieden bei jedem Auftreten NR¹, O, S, Se, Te, SO, SeO, TeO, SO₂, SeO₂ oder TeO₂;
- Z: hat dieselbe Bedeutung, wie oben für V beschrieben;
- c: ist gleich oder verschieden bei jedem Auftreten 0 oder 1.

Darüberhinaus sind ebenfalls bevorzugt die Verbindungen (9) bis (12), gemäß Schema 2, die jeweils noch einen oder zwei zusätzliche Liganden L3 tragen können, wie oben beschrieben. 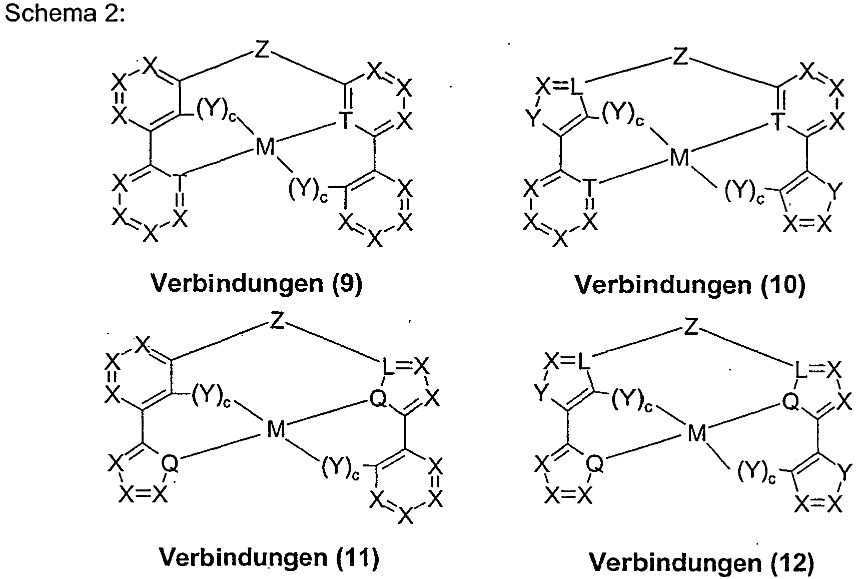 wobei die Symbole und Indizes M, L, Q, T, X, Y, Z, R, R¹, R² und c die oben angegebene Bedeutung haben.

Ein weiterer Gegenstand der Erfindung sind Verbindungen, die gleichzeitig Liganden vom Typ wie bei Verbindungen (1), (2), (3) und/oder (4) aufweisen, d. h. gemischte Ligandensysteme. Diese werden durch die Formeln (13) bis (30) - gemäß Schema 3 - beschrieben, die jeweils noch einen oder zwei zusätzliche Liganden L3 tragen können, wie oben beschrieben: 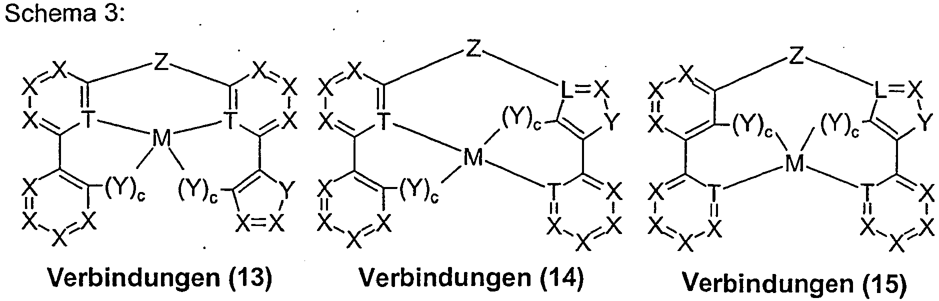 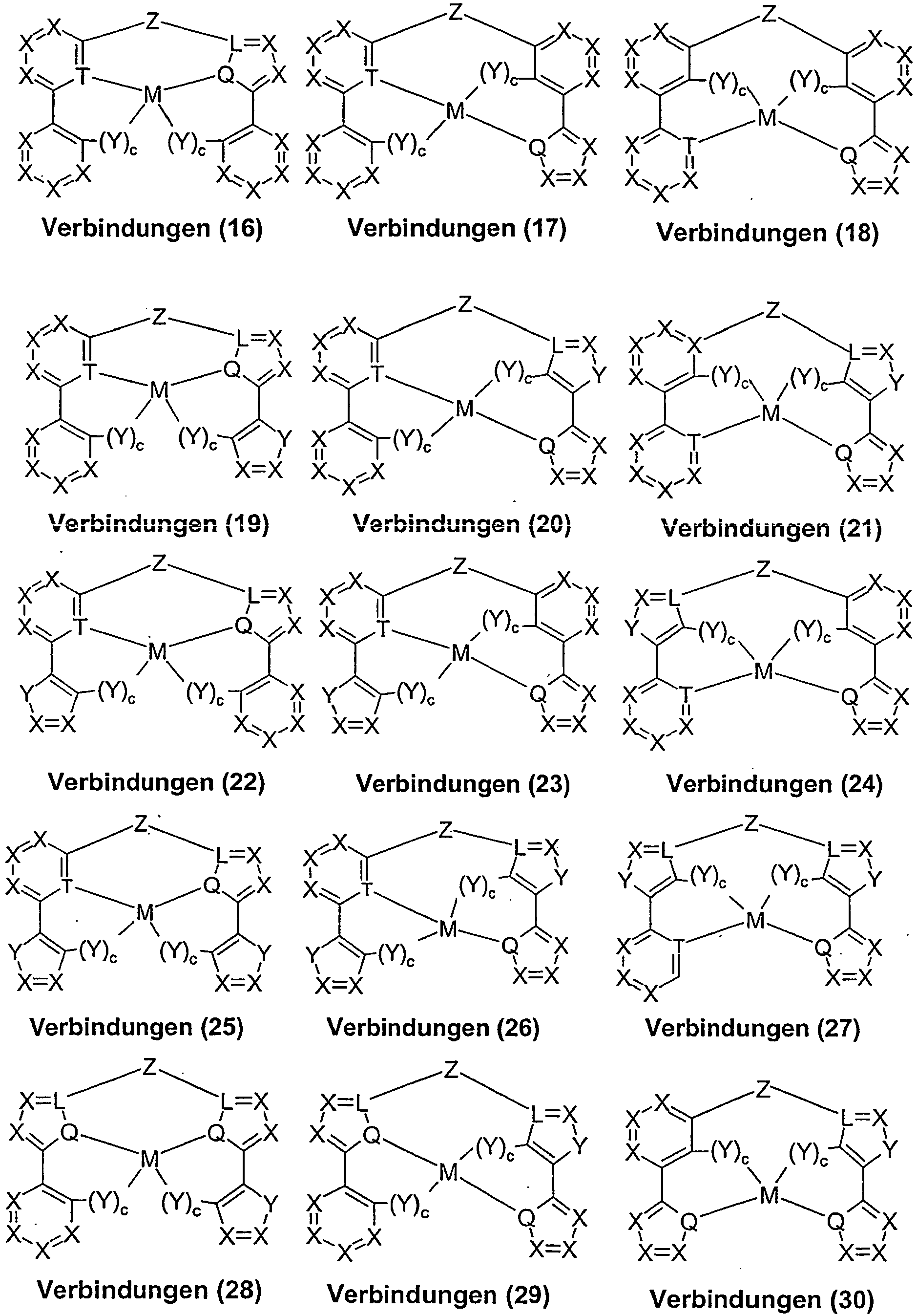 wobei die Symbole und Indizes M, L, Q, T, X, Y, Z, R, R¹, R² und c die oben angegebene Bedeutung haben.

Gegebenenfalls können die Verbindungen der Struktur 1 bzw. die Verbindungen (1) bis (30) weitere ein- oder mehrzähnige, kationische, neutrale oder anionische Liganden tragen, wie oben bereits beschrieben. Diese werden durch den Liganden L3 beschrieben.

Bevorzugt sind Verbindungen der Struktur 1 bzw. die Verbindungen (1) bis (30), dadurch gekennzeichnet, daß der Teilligand L3, wenn vorhanden, ein zweizähnig chelatisierender Ligand ist.

In einer bevorzugten Ausführungsform der Erfindung ist L3 ein monoanionischer Ligand gleich oder verschieden den Teilliganden L1 bzw. L2.

In einer weiteren bevorzugten Ausführungsform der Erfindung ist L3 ein Ligand gemäß Struktur (4), 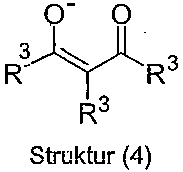 wobei R³ gleich oder verschieden bei jedem Auftreten H, eine C₁ bis C₂₀ Alkylgruppe, eine C₁ bis C₂₀ Alkoxygruppe, eine C₄ bis C₂₀ Aryl- oder Heteroarylgruppe oder eine C₄ bis C₂₀ Aryloxy- oder Heteroaryloxygruppe darstellt und ein oder mehrere H-Atome durch F ersetzt sein können.

Bevorzugt sind erfindungsgemäße Verbindungen (1) bis (30), bei denen für das Symbol M = Be, Mg, Pt oder Zn gilt und der Index a = 0 ist.

Besonders bevorzugt sind erfindungsgemäße Verbindungen (1) bis (30), bei denen c = 0 und M = Pt gilt.

Weiterhin bevorzugt sind erfindungsgemäße Verbindungen (1) bis (30), bei denen für das Symbol M = Rh oder Ir, besonders bevorzugt M = Ir, gilt und der Index a = 1 mit einem zweizähnigen monoanionischen Liganden L3 oder a = 2 mit einzähnigen monoanionischen Liganden L3 ist.

Bevorzugt sind ebenfalls erfindungsgemäße Verbindungen (1) bis (30), bei denen für das Symbol L = C oder N gilt, besonders bevorzugt L = C.

Bevorzugt sind ebenfalls erfindungsgemäße Verbindungen (1) bis (30), bei denen für das Symbol Q = O oder S gilt.

Bevorzugt sind ebenfalls erfindungsgemäße Verbindungen (1) bis (30), bei denen für das Symbol T = N gilt.

Bevorzugt sind ebenfalls erfindungsgemäße Verbindungen (1) bis (30), bei denen für das Symbol X = CR oder N gilt.

Bevorzugt sind ebenfalls erfindungsgemäße Verbindungen (1) bis (30), bei denen für das Symbol Z = CR₂, CO, SiR¹₂, R¹N, FP, FPO, R¹P, R¹PO, -CR₂CR₂-, -CR₂-O-CR₂-, -O-(OR¹)PO-O-, cis-CR=CR, -CR₂-BR¹-CR₂-, -CR₂-CO-CR₂-, -CR₂-CR₂-CR₂- oder - CR₂-NR¹-CR₂ gilt.

Bevorzugt sind ebenfalls erfindungsgemäße Verbindungen (1) bis (30), bei denen für das Symbol R = H, F, Cl, Br, I, CN, eine geradkettige, verzweigte oder cyclische Alkyl- oder Alkoxygruppe mit 1 bis 6 C-Atomen oder eine Aryl- oder Heteroarylgruppe mit 3 bis 10 C-Atomen, die durch einen oder mehrere nicht aromatische Reste R substituiert sein kann, wobei mehrere Substituenten R, sowohl am selben Ring als auch an den beiden unterschiedlichen Ringen zusammen wiederum ein weiteres mono- oder polycyclisches, aliphatisches oder aromatisches Ringsystem aufspannen können, gilt.

In der Verbindungen (1) bis (30) können durch die Reste R aliphatische, olefinische oder aromatische Ringsysteme aufgespannt werden.

Sofern in den Verbindungen (1) bis (30) durch die Reste R aromatische Ringsysteme aufgespannt werden, sind diese bevorzugt Benzen, 1- bzw. 2-Naphthalin, 1-, 2- bzw. 9-Anthracen, 2-, 3- bzw. 4-Pyridin, 2-, 4- bzw. 5-Pyrimidin, 2-Pyrazin, 3- bzw. 4-Pyridazin, Triazin, 2-, 3-, 4-, 5-, 6-, 7- bzw. 8-Chinolin, 2- bzw. 3-Pyrrol, 3-, 4-, 5-Pyrazol, 2-, 4-, 5-Imidazol, 2-, 3-Thiophen, 2-, 3-Selenophen, 2- bzw. 3-Furan, 2-(1,3,4-Oxadiazol), Indol und Carbazol.

Die erfindungsgemäßen Verbindungen zeichnen sich durch folgende generelle Eigenschaften aus:
1. Die erfindungsgemäßen Verbindungen weisen - im Gegensatz zu vielen bekannten Metallkomplexen, die der teilweisen oder vollständigen pyrolytischen Zersetzung bei Sublimation unterliegen - eine große thermische Stabilität auf. Diese gilt besonders für die erfindungsgemäßen Platin- und Iridiumkomplexe vierzähnig chelatisierender Liganden, die neben dativer Koordination über ein Heteroatom auch wenigstens eine Arylkohlenstoff-Platin- bzw. -Iridium-Bindung enthalten. Die hohe Stabilität der erfindungsgemäßen Verbindungen führt bei Verwendung in entsprechenden Vorrichtungen zu einer deutlichen Erhöhung der operativen Lebensdauer.
2. Die erfindungsgemäßen Verbindungen weisen keine erkennbare Hydrolyse bzw. Hygroskopie auf. Lagerung für mehrere Tage bzw. Wochen unter Zutritt von Luft und Wasserdampf führt zu keinen Veränderungen der Substanzen. Die Anlagerung von Wasser an die Verbindungen konnte nicht nachgewiesen werden. Dies hat den Vorteil, daß die Substanzen unter einfacheren Bedingungen gereinigt, transportiert, gelagert und für den Einsatz vorbereitet werden können.
3. Die erfindungsgemäßen Verbindungen - eingesetzt als Elektronentransportmaterial in Elektrolumineszenz-Vorrichtungen - führen in diesen zu hohen Effizienzen, die insbesondere unabhängig von den verwendeten Stromdichten sind. Damit werden auch bei hohen Stromdichten, d. h. hohen Helligkeiten, sehr gute Effizienzen erreicht.
4. Die erfindungsgemäßen Verbindungen - eingesetzt als Lochblockiermaterial in Elektrolumineszenz-Vorrichtungen - führen in diesen zu hohen Effizienzen, die insbesondere unabhängig von den verwendeten Stromdichten sind. Damit werden auch bei hohen Stromdichten sehr gute Effizienzen erreicht. Außerdem sind die erfindungsgemäßen Materialien stabil gegen Löcher, was bei anderen Metallkomplexen, z. B. AIQ₃ und analogen Verbindungen, nicht in ausreichendem Maße gegeben ist (Z. Popovic et al., Proceedings of SPIE, 1999, 3797,310-315).
5. Die erfindungsgemäßen Verbindungen - eingesetzt in Elektrolumineszenz-Vorrichtungen als Emissionsmaterial in reiner Form oder als Emissionsmaterial dotiert in ein Matrixmaterial oder als Matrixmaterial in Kombination mit einem Dotanden - führen zu hohen Effizienzen, wobei sich die Elektrolumineszenz-Vorrichtungen durch steile Strom-Spannungs-Kurven und besonders durch lange operative Lebensdauern auszeichnen.
6. Die erfindungsgemäßen Verbindungen sind durch die strukturellen Vorgaben so gestaltet, daß sie nicht planar sind und damit eine Aggregation unter Ausbildung Austauschseite 13
   1. starker Metall-Metall-, Metall-Ligand- oder Ligand-Ligand-Wechselwirkungen unterdrückt wird.
   2. Die Unterdrückung der Aggregation dieser Verbindungen führt zum einen zu schmalen Emissionsbanden und damit reineren Emissionsfarben. Zum anderen ist die Emissionsfarbe über weite Bereiche unabhängig vom Dotierungsgrad, was für technische Anwendungen von großem Vorteil ist.
   3. Ohne an eine bestimmte Theorie gebunden sein zu wollen, ist der strukturell starre Aufbau der erfindungsgemäßen Verbindungen hohen Quanteneffizienzen der Emissionsübergänge förderlich.
   4. Die erfindungsgemäßen Verbindungen sind gut reproduizerbar in verläßlich hoher Reinheit herstellbar und weisen keine Chargenschwankung auf.
   5. Die erfindungsgemäßen Verbindungen weisen zum Teil exzellente Löslichkeit in organischen Lösungsmitteln auf, wobei die Löslichkeit durch eine geeignete Wahl des Substitutionsmusters, z. B. durch Einführung verzweigter Alkylketten in der Brücke V bzw. Z, maßgeschneidert werden kann. Damit sind diese Materialien auch aus Lösung durch Beschichtungs- oder Drucktechniken verarbeitbar. Auch bei der üblichen Verarbeitung durch Verdampfung ist diese Eigenschaft von Vorteil, da so die Reinigung der Anlagen bzw. der eingesetzten Schattenmasken durch Waschen erheblich erleichtert wird.

Die Verbindungen (31) bis (60) gemäß Schema 4 stellen die Liganden der erfindungsgemäßen Verbindungen gemäß Struktur 1 dar und sind somit wertvolle Zwischenprodukte auf dem Weg zu diesen Verbindungen: 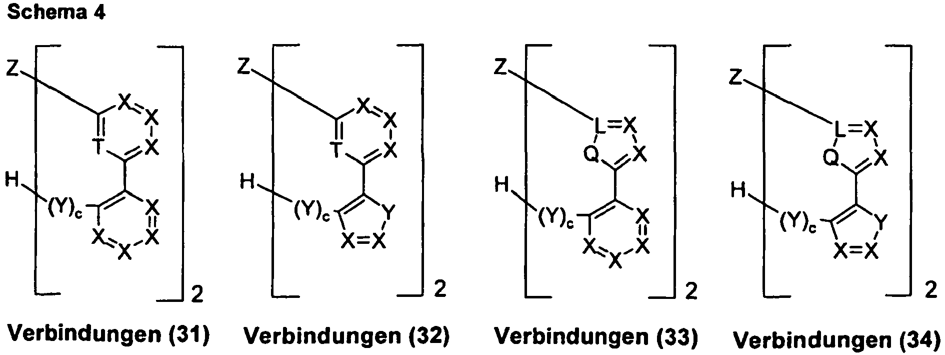 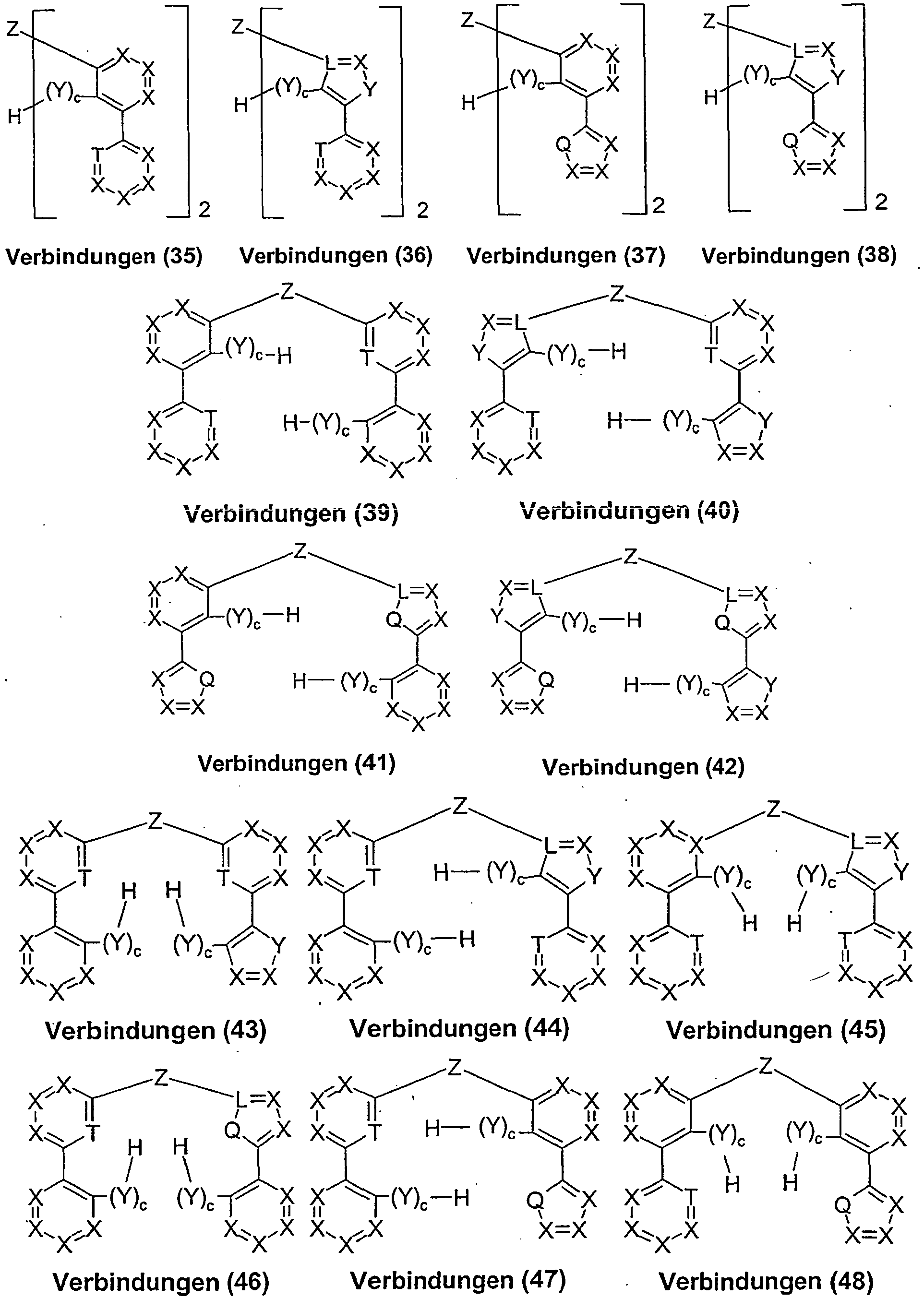 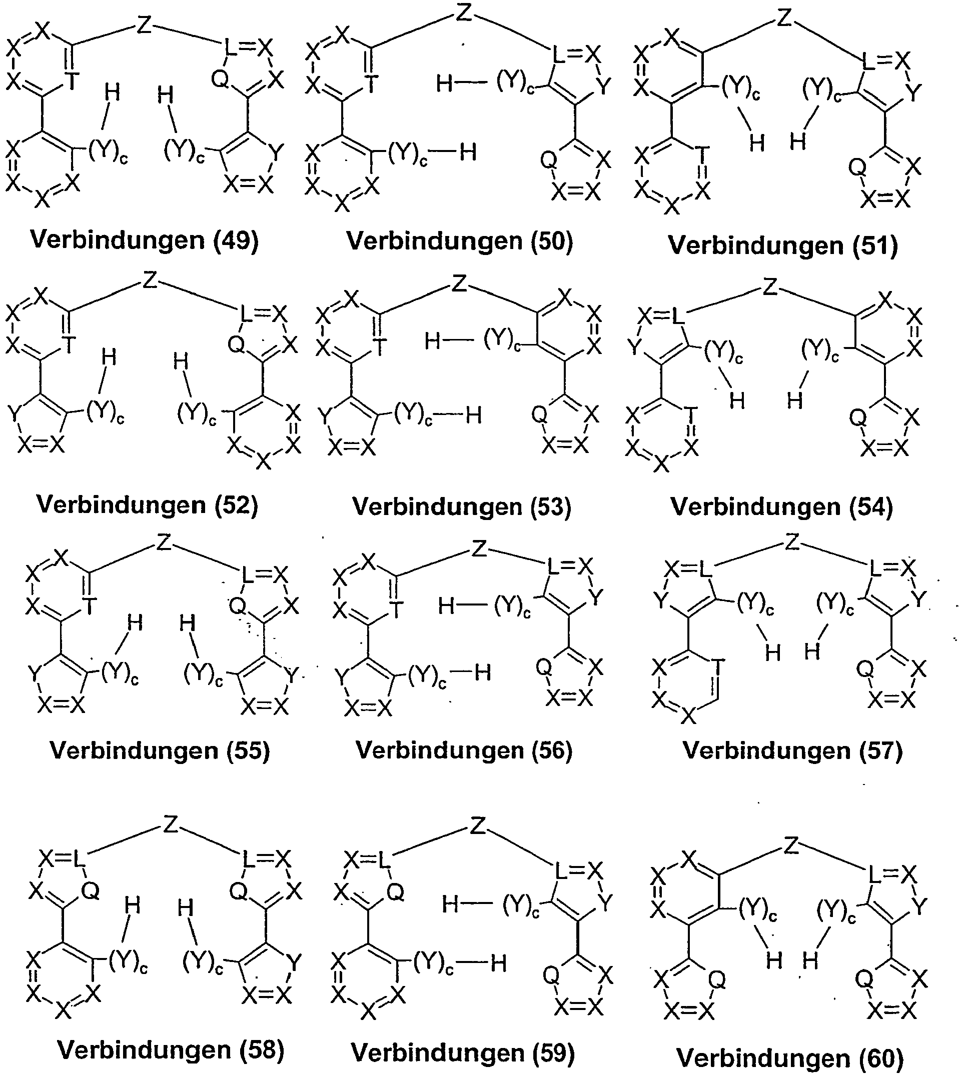 wobei die Symbole und Indizes Q, L, T, X, Y, Z, R, R¹, R² und c die oben angegebene Bedeutung haben, ausgenommen die Verbindungen Bis(6-phenyl-2-pyridyl)methan [CAS 362602-93-5], Bis(6-phenyl-2-pyridyl)keton [CAS 217177-35-0], Bis(6-(1-hydroxy-3,5-di-tert-butyl)phenyl-2-pyridyl)methanol [CAS 367525-74-4], 2,2'-Thio-bis(3-cyano-2,4-diphenyl]pyridin [CAS 160598-76-5], Bis(6-(3-phenyl)phenyl-2-pyridyl)methan [CAS 57476-80-9] und Isomere [CAS 57476-79-6].

Die vorstehenden Verbindungen (31) bis (60) sind bereits in Struktur 2 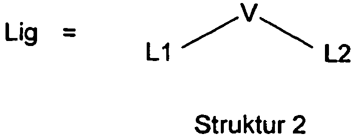 eingehend beschrieben und folgen dem gleichen Konzept (V = Z).

Die Verbindungen (31) bis (60) sind durch gängige organische Reaktionen darstellbar, was im folgenden an ausreichend vielen Beispielen belegt wird. So können die Verbindungen (31) ausgehend von Di(6-brom-2-pyridyl)keton (WO 98/22148) mit aliphatischen oder aromatischen Lithium- oder Grignardreagenzien umgesetzt werden, wobei ein Dipyridylmethanol entsteht. Dieses kann dann z. B. durch Umsetzung mit Halogenierungsagenzien wie Diethylaminoschwefeltrifluorid (DAST) fluoriert, mit Thionylchlorid chloriert oder mit Phosphortribromid bromiert werden. Eine Alkylierung der Hydroxylgruppe unter Bildung eines Ethers ist ebenfalls leicht durchführbar. Eine abschließende Suzuki-Kupplung mit Arylboronsäuren führt dann zu den Verbindungen (31). Diese Reaktionssequenz ist an einem konkreten Beispiel - Methylierung, Fluorierung, Kupplung mit Phenylboronsäure - in Schema 5 dargestellt und führt zu Verbindungen (31) mit c = 0.

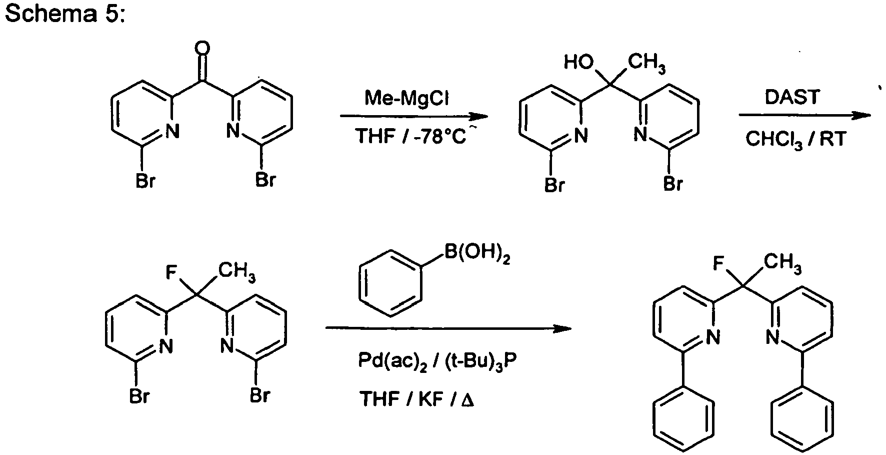

Eine analoge Reaktionssequenz unter Verwendung von Tetrahydropyranylgeschützten Phenolboronsäuren, die aus den entsprechenden Bromphenolen durch Schützen mit Dihydropyran, anschließende Grignardreaktion und Umsetzung mit einem Borsäureester dargestellt werden können, führt nach Schutzgruppenabspaltung zu Verbindungen des Typs (31) mit c = 1 (Schema 6).

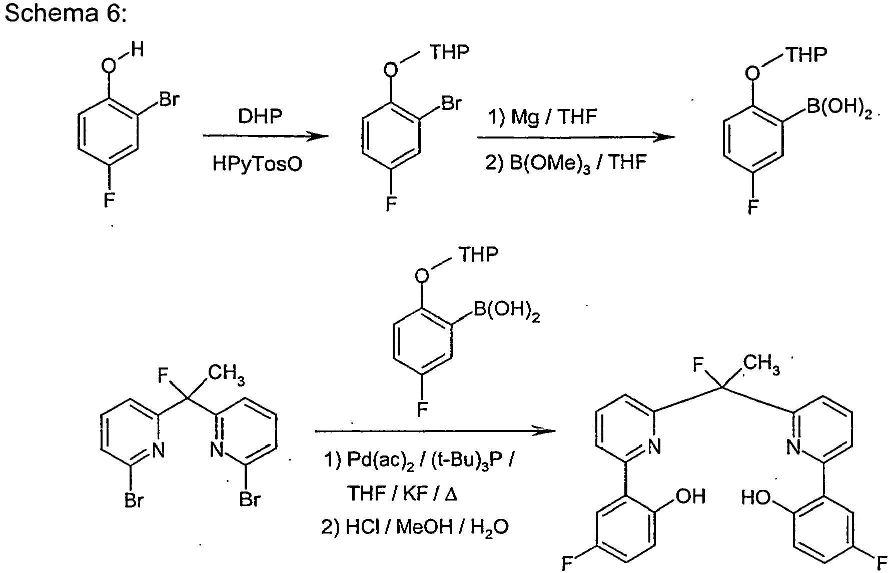

In analoger Weise sind auch die Verbindungen (32) bis (38) durch Verwendung der entsprechenden 5- und 6-gliedrigen Heterocylen darstellbar.

Verbindungen des Typs (39) und (40) können z. B. nach der in Schema 7 an einem konkreten Beispiel aufgezeigten Reaktionssequenz dargestellt werden. Selbstverständlich können auch hier durch die Variation der Edukte (Arylhalogenide bzw. Boronsäuren) eine Vielzahl weiterer Verbindungen erhalten werden. 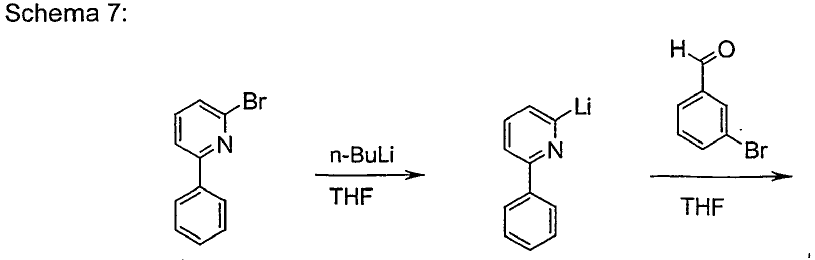 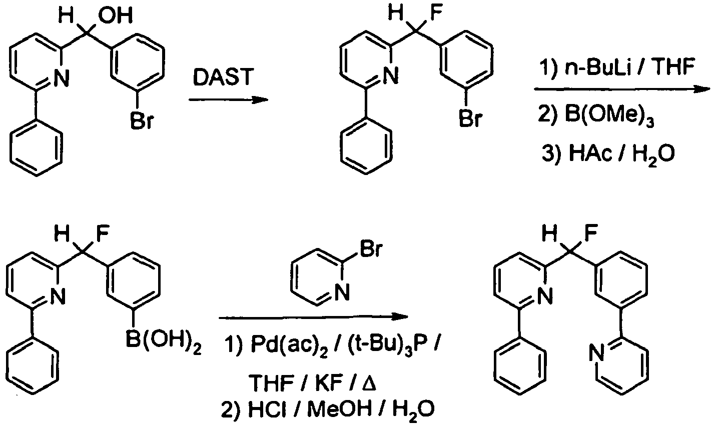 Abschließend sei bemerkt, daß in völlig analoger Weise durch Verwendung analoger Reaktionssequenzen auch die Verbindungen (41) bis (60) zugänglich sind.

Ausgehend von 2-Lithio-6-phenylpyridin (Gros et al., J. Org. Chem., 2003, 68(5), 2028-2029) und dessen Analoga lassen sich Liganden, welche Heteroatome in der Brücke V bzw. Z tragen, darstellen, wobei als weitere Synthone geeignete Elektrophile, die das Heteroatom enthalten, verwendet werden können. Als Elektrophile kommen unter anderem Dichlor-arylborane, Dichlor-alkyl- oder -arylsilane bzw. Dichlor-aryl- oder alkylphosphine in Frage, wie in Schema 8 gezeigt.

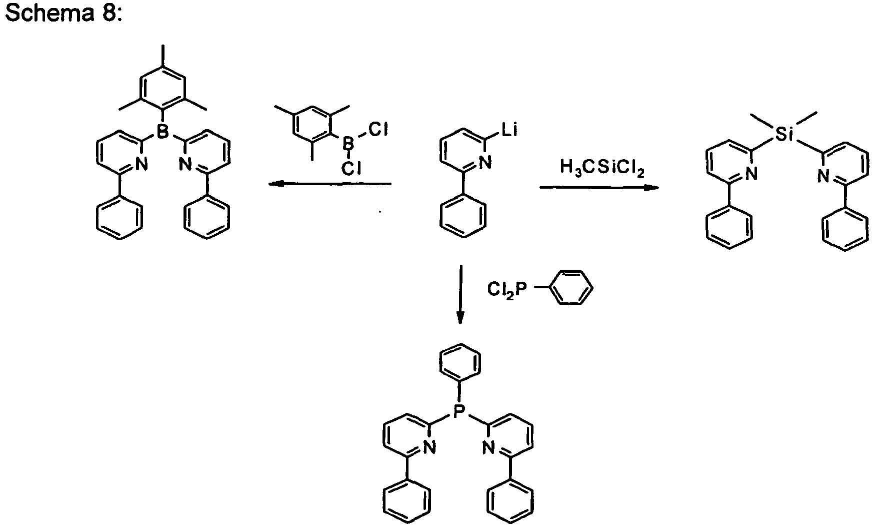

Die erfindungsgemäßen Verbindungen (1) bis (30) sind prinzipiell durch verschiedene Verfahren herstellbar; es haben sich jedoch die im folgenden beschriebenen neuen Verfahren als besonders gut geeignet herausgestellt.

Daher ist ein weiterer Gegenstand der vorliegenden Erfindung ein Verfahren zur Herstellung der Verbindungen (1) bis (30) durch Umsetzung der vierzähnig chelatisierenden Liganden gemäß Verbindungen (31) bis (60) mit Metallalkoholaten der Verbindung (61), mit Metallketoketonaten der Verbindung (62), Metallhalogeniden, -carboxylaten, -nitraten, -sulfaten der Verbindung (63) und Metallalkyl- bzw. -arylverbindungen der Verbindung (64), 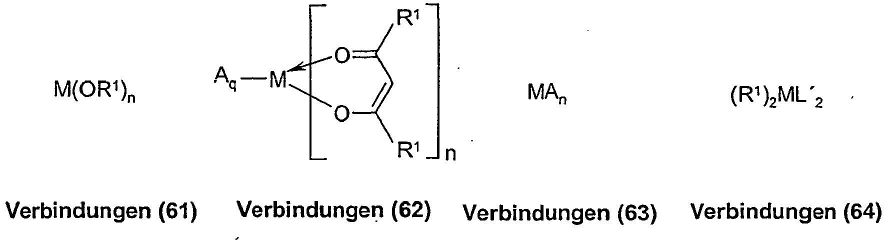 wobei die Symbole M und R¹ die unter Schema 1 angegebene Bedeutung haben und A = F, Cl, Br, I, OH, Formiat, Acetat, Propionat, Benzoat, Nitrat oder Sulfat und L' ein einzähniger Ligand aus der Gruppe der Ether, wie z. B. THF, der Amine, wie z. B. Trimethylamin oder Pyridin, der Phosphine, wie z. B. Triphenylphosphin oder der Sulfoxide, wie z. B. DMSO, und n = 1, 2 oder 3 und q = 0, 1, 2 oder 3, bevorzugt 0, 1 oder 2, ist. Dabei kann die Verbindung (62) auch geladen sein. Gegebenenfalls können als Hilfsagenzien Lewissäuren, wie z. B. Aluminiumchlorid oder Antimonpentafluorid oder -chlorid, oder Brönstedbasen, wie z. B. Amine, oder Alkylierungsmittel, wie z. B. Organolithium- oder Grignardverbindungen, zugesetzt werden.

Weiterhin kann es vorteilhaft sein, die Reaktion in mehreren Einzelschritten zur Einführung der einzelnen Liganden durchzuführen. So kann es beispielsweise bevorzugt sein, wenn zunächst der Ligand Lig eingeführt wird und der Komplex noch weitere Hilfsliganden (beispielsweise Halogenide) enthält, die dann in einem weiteren Schritt gegen einen zweizähnig chelatisierenden Liganden L3 ausgetauscht werden. Ebenso können beispielsweise erst die Teilligangen L1 und L2 in den Komplex eingeführt werden, die dann in einem Folgeschritt mit der verbrückenden Einheit V bzw. Z verknüpft werden.

Dadurch lassen sich die Verbindungen (1) bis (30) in hoher Reinheit, bevorzugt > 99 % (bestimmt mittels ¹H-NMR und/oder HPLC), erhalten.

Mit den hier erläuterten Synthesemethoden lassen sich unter anderem die im folgenden dargestellten Beispiele für die Verbindungen (1) bis (30) herstellen.

| | | |
|---|---|---|
| 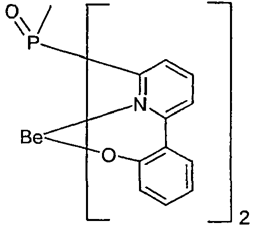 | 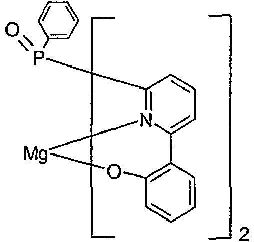 | 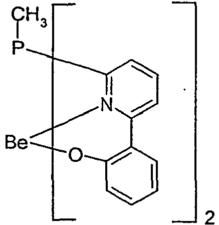 |
| Beispiel 1 | Beispiel 2 | Beispiel 3 |
| 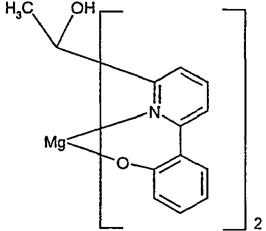 | 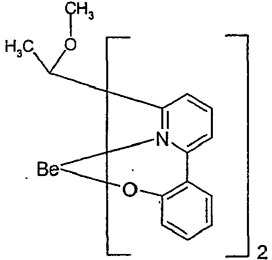 | 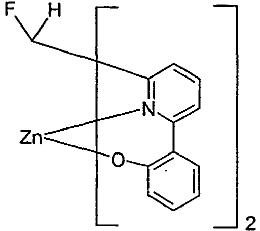 |
| Beispiel 4 | Beispiel 5 | Beispiel 6 |
| 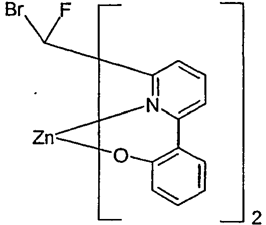 | 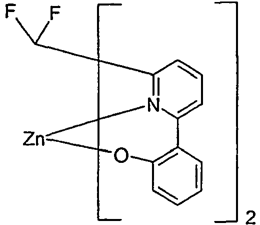 | 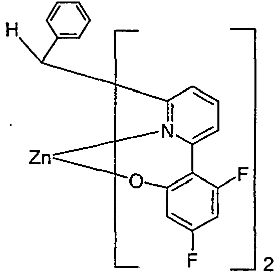 |
| Beispiel 7 | Beispiel 8 | Beispiel 9 |
| 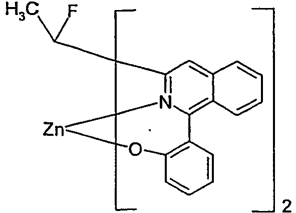 | 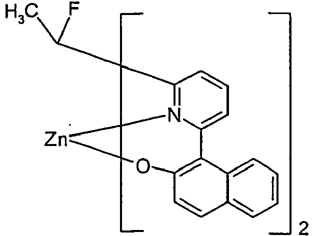 | 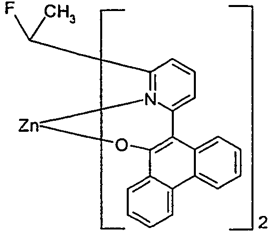 |
| Beispiel 10 | Beispiel 11 | Beispiel 12 |
| 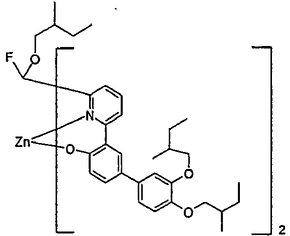 | 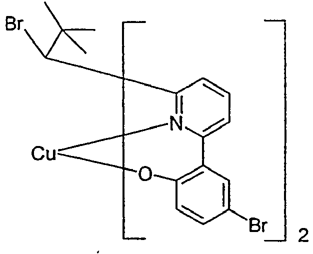 | 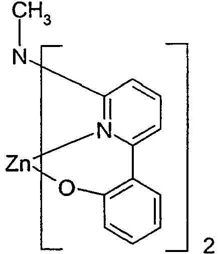 |
| Beispiel 13 | Beispiel 14 | Beispiel 15 |
| 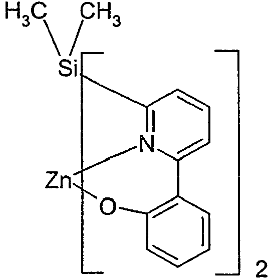 | 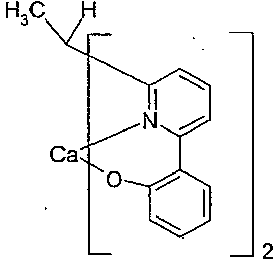 | 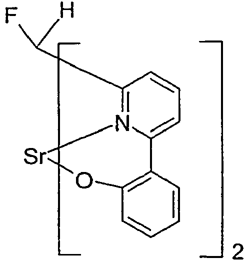 |
| Beispiel 16 | Beispiel 17 | Beispiel 18 |
| 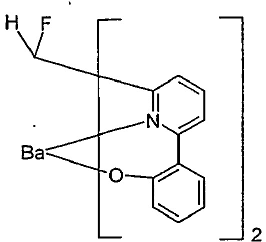 | 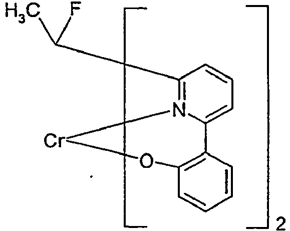 | 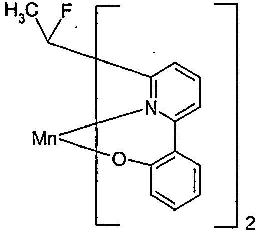 |
| Beispiel 19 | Beispiel 20 | Beispiel 21 |
| 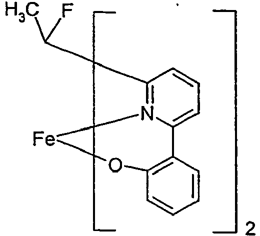 | 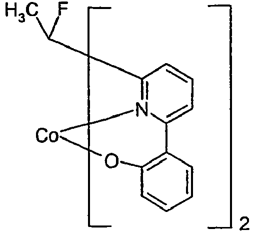 | 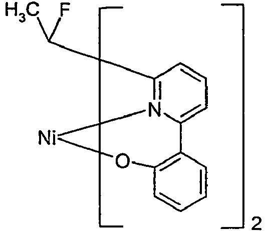 |
| Beispiel 22 | Beispiel 23 | Beispiel 24 |
| 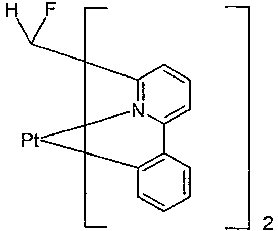 | 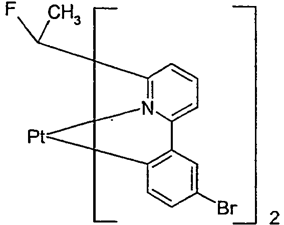 | 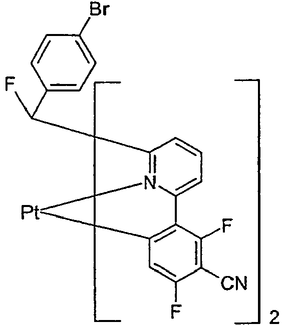 |
| Beispiel 25 | Beispiel 26 | Beispiel 27 |
| 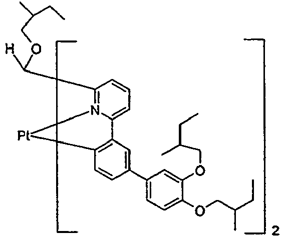 | 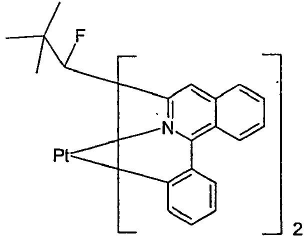 | 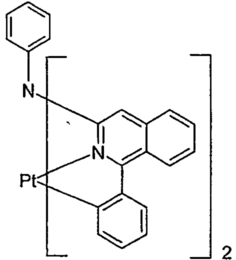 |
| Beispiel 28 | Beispiel 29 | Beispiel 30 |
| 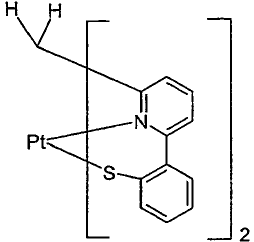 | 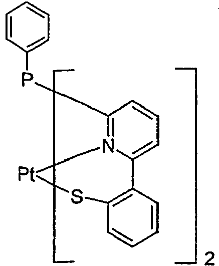 | 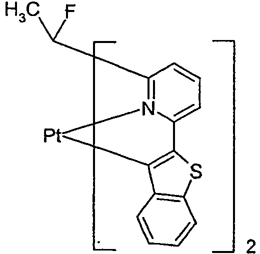 |
| Beispiel 31 | Beispiel 32 | Beispiel 33 |
| 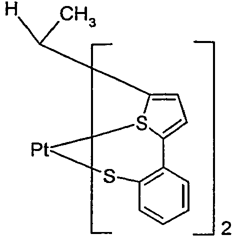 | 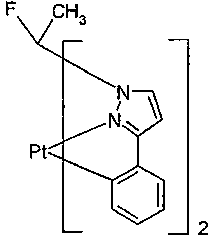 | 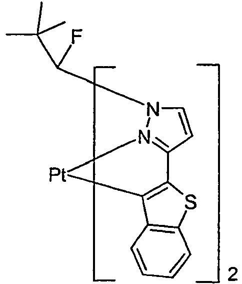 |
| Beispiel 34 | Beispiel 35 | Beispiel 36 |
| 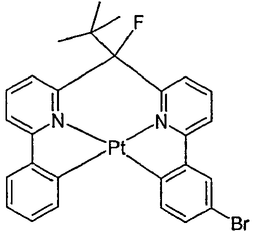 | 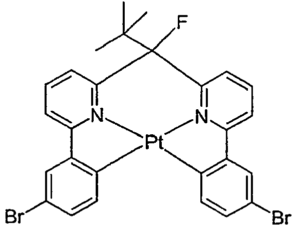 | 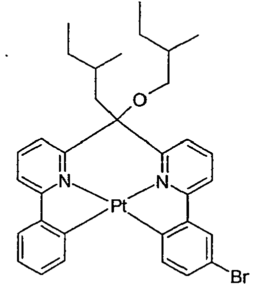 |
| Beispiel 37 | Beispiel 38 | Beispiel 39 |
| 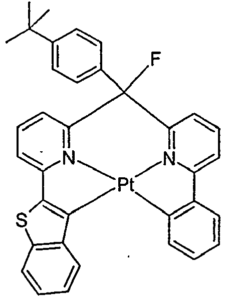 | 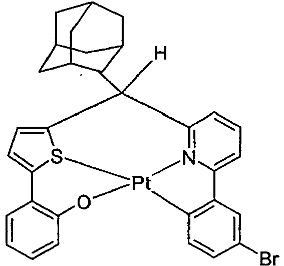 | 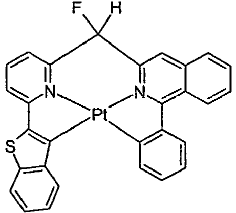 |
| Beispiel 40 | Beispiel 41 | Beispiel 42 |
| 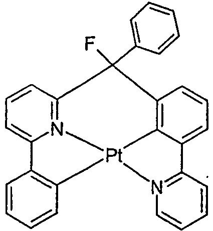 | 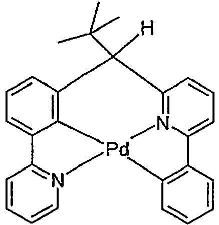 | 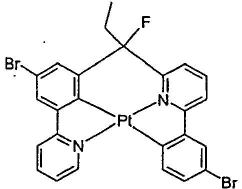 |
| Beispiel 43 | Beispiel 44 | Beispiel 45 |
| 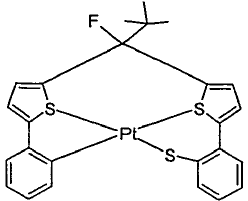 | 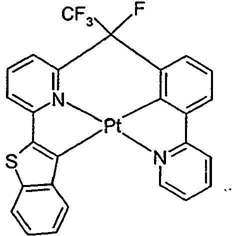 | 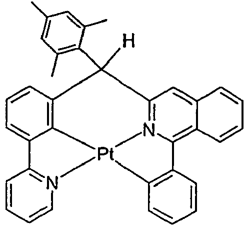 |
| Beispiel 46 | Beispiel 47 | Beispiel 48 |
| 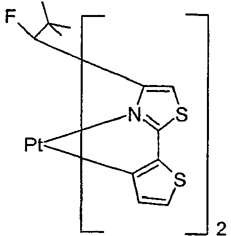 | 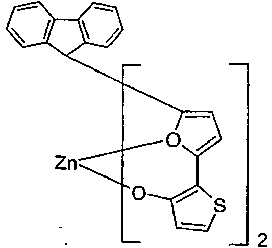 | 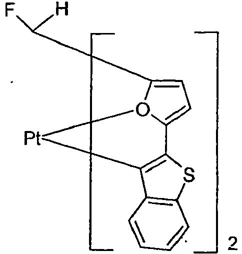 |
| Beispiel 49 | Beispiel 50 | Beispiel 51 |
| 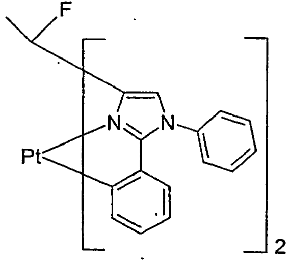 | 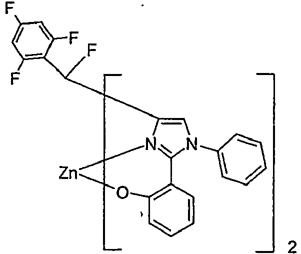 | 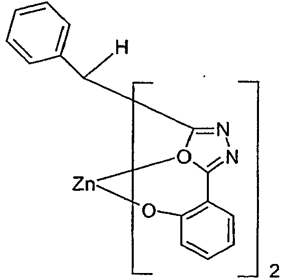 |
| Beispiel 52 | Beispiel 53 | Beispiel 54 |
| 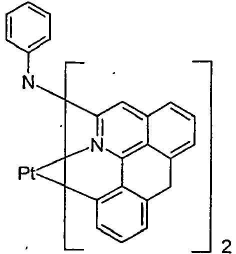 | 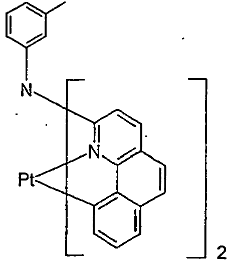 | 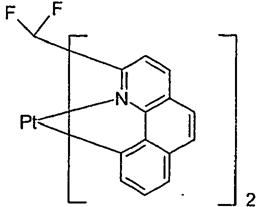 |
| Beispiel 55 | Beispiel 56 | Beispiel 57 |
| 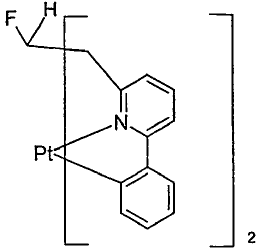 | 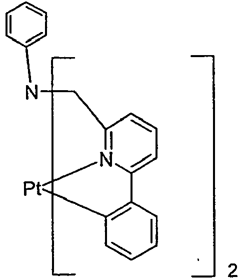 | 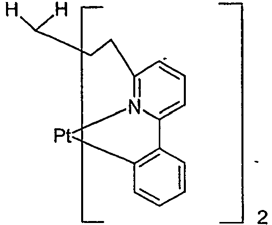 |
| Beispiel 58 | Beispiel 59 | Beispiel 60 |
| 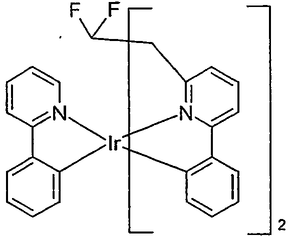 | 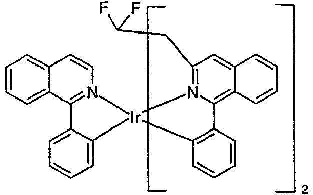 | 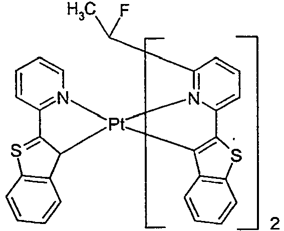 |
| Beispiel 61 | Beispiel 62 | Beispiel 63 |
| 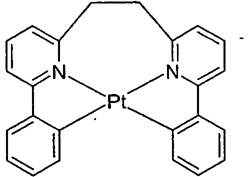 | 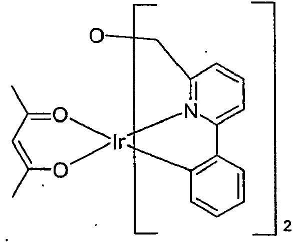 | 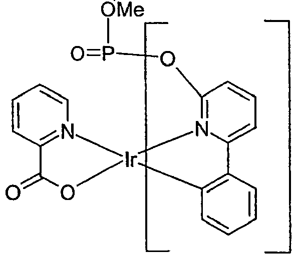 |
| Beispiel 64 | Beispiel 65 | Beispiel 66 |

Generell gelten auch Strukturen als erfindungsgemäß, die obige Strukturelemente als Substrukturen enthalten, beispielsweise die Verbindungen gemäß Schema 9.

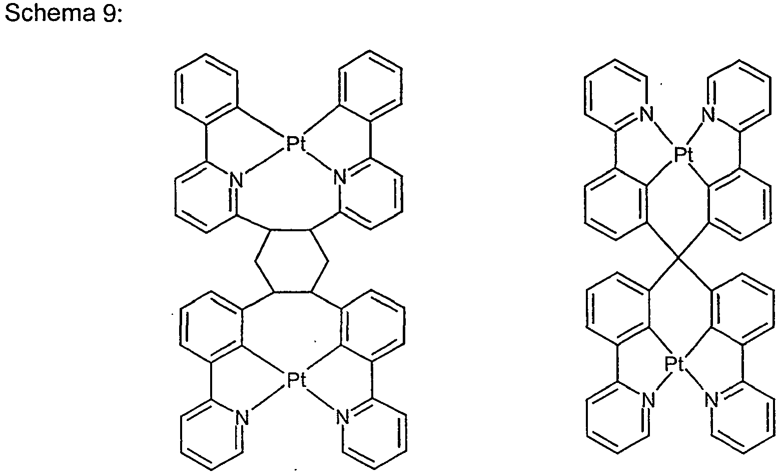

Die oben beschriebenen erfindungsgemäßen Verbindungen - z. B. Verbindungen gemäß den Beispielen 7, 14, 26, 27, 37, 38, 39, 41, 45 - können auch als Comonomere zur Erzeugung entsprechender konjugierter, teilkonjugierter oder nicht-konjugierter Polymere oder Dendrimere - z. B. Verbindungen gemäß den Beispielen 14 und 26 - Verwendung finden. Die entsprechende Polymerisation erfolgt dabei bevorzugt über die Halogenfunktionalität. So können sie u. a. in lösliche Polyfluorene (z. B. gemäß EP 842208 oder WO 00/22026), Poly-spirobifluorene (z. B. gemäß EP 707020 oder EP 894107), Poly-para-phenylene (z. B. gemäß WO 92/18552), Poly-dihydrophenanthrene (z. B. gemäß DE 10337346.2), Poly-indenofluorene (z. B. gemäß WO 04/041901 oder EP 03014042.0), Poly-carbazole (z. B. gemäß DE10304819.7 oder DE10328627.6), Polythiophene (z. B. gemäß EP 1028136), Polyvinylcarbazole oder auch Polyketone einpolymerisiert werden oder auch in Copolymere aus zwei oder mehreren dieser Einheiten.

Weiterer Gegenstand der Erfindung sind somit konjugierte, teilkonjugierte oder nicht-konjugierte Polymere oder Dendrimere, enthaltend eine oder mehrere Verbindungen (1) bis (30), wobei mindestens einer der oben definierten Reste R eine Bindung zum Polymer oder Dendrimer darstellt.

Weiterhin können die erfindungsgemäßen Metallkomplexe auch durch die beispielsweise o. g. Reaktionstypen weiter funktionalisiert werden und so zu erweiterten Metallkomplexen umgesetzt werden. Hier ist als Beispiel die Funktionalisierung mit Arylboronsäuren gemäß SUZUKI oder mit Aminen gemäß HARTWIG-BUCHWALD zu nennen.

Die oben beschriebenen erfindungsgemäßen Verbindungen (1) bis (30), die Polymere und Dendrimere enthaltend als Comonomere Verbindungen des Typs (1) bis (30) und die erweiterten Metallkomplexe finden Verwendung als aktive Komponenten in elektronischen Bauteilen, wie z. B. Organischen Leuchtdioden (OLEDs), Organischen Integrierten Schaltungen (O-ICs), Organischen Feld-Effekt-Transistoren (OFETs), Organischen Dünnfilmtransistoren (OTFTs), Organischen Solarzellen (O-SCs) oder auch Organischen Laserdioden (O-Laser).

Gegenstand der Erfindung ist also auch die Verwendung der oben beschriebenen erfindungsgemäßen Verbindungen (1) bis (30), der Polymere und Dendrimere enthaltend als Comonomere Verbindungen des Typs (1) bis (30) und der erweiterten Metallkomplexe in elektronischen und/oder optischen Vorrichtungen, wie z. B. Organischen Leuchtdioden (OLEDs), Organischen Integrierten Schaltungen (O-ICs), Organischen Feld-Effekt-Transistoren (OFETs), Organischen Dünnfilmtransistoren (OTFTs), Organischen Solarzellen (O-SCs) oder auch Organische Laserdioden (O-Laser).

Gegenstand der Erfindung sind weiterhin elektronische und/oder optische Vorrichtungen, insbesondere Organische Leuchtdioden (OLEDs), Organische Integrierte Schaltungen (O-ICs), Organische Feld-Effekt-Transistoren (OFETs), Organische Dünnfilmtransistoren (OTFTs), Organische Solarzellen (O-SCs) oder auch Organische Laserdioden (O-Laser), enthaltend eine oder mehrere der erfindungsgemäßen Verbindungen (1) bis (30), der Polymere und Dendrimere enthaltend als Comonomere Verbindungen des Typs (1) bis (30) und der erweiterten Metallkomplexe.

Die vorliegende Erfindung wird durch die folgenden Beispiele näher erläutert, ohne sie darauf beschränken zu wollen. Der Fachmann kann aus den Schilderungen ohne erfinderisches Zutun weitere erfindungsgemäße Komplexe herstellen bzw. das erfindungsgemäße Verfahren anwenden.

Die OLEDs enthaltend eine oder mehrere der erfindungsgemäßen Verbindungen können nach dem Fachmann geläufigen Verfahren, wie z. B. in WO 04/058911 und DE 10317556.3 beschrieben, hergestellt werden.

### Beispiele:

Die nachfolgenden Synthesen wurden, sofern nicht anders angegeben, unter einer Schutzgasatmosphäre in getrockneten Lösungsmitteln durchgeführt. Die Edukte wurden von ALDRICH bzw. ABCR [Methylmagnesiumchlorid 3 M in THF, Diethylaminoschwefeltrifluorid (DAST), Benzolboronsäure, Kaliumfluorid (sprühgetrocknet), Tri-*tert*-butylphosphin, Palladium(II)acetat, Kaliumtetrachloroplatinat] bezogen. Di(6-brom-2-pyridyl)keton wurde, wie in WO 98/22148 beschrieben, dargestellt. *cis*-Dimethyl-di(η¹-S-dimethylsulfoxidyl)platin(II) wurde nach C. Eaborn et al., J. Chem. Soc., Dalton Trans., 1981, 933-938 dargestellt.

### Ligandesnsynthese

### Beispiel 1: 1,1-Bis(6-phenyl-2-pyridyl)-1-fluorethan

### a) 1,1-Bis(6-brom-2-pyridyl)ethan-1-ol

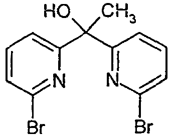

Zu einer auf -78 °C gekühlten Suspension von 102.6 g (300 mmol) Di(6-brom-2-pyridyl)keton in 1000 ml THF wurden unter gutem Rühren 113 ml (340 mmol) einer 3 M Methylmagnesiumchlorid-Lösung in THF so zugetropft, daß eine Temperatur von -60 °C nicht überschritten wurde. Nach vollendeter Zugabe wurde noch 30 min. nachgerührt, dann wurden 50 ml Ethanol zugetropft und nach Erwärmen auf 0 °C mit 60 ml halbgesättigter Ammoniumchloridlösung versetzt. Die Reaktionsmischung wurde filtriert, die Salze wurden zweimal mit je 100 ml THF gewaschen und das Filtrat wurde am Rotationsverdampfer zur Trockene eingeengt. Der ölige Rückstand wurde in 1000 ml Dichlormethan aufgenommen, die organische Phase wurde dreimal mit 300 ml Wasser gewaschen und über Magnesiumsulfat getrocknet. Nach Abziehen des Dichlormethans verblieben 106.0 g (296 mmol), entsprechend einer Ausbeute von 98.6 %, des Rohprodukts mit einer Reinheit von ca. 95% nach ¹H-NMR als gelbbraunes Öl, welches ohne Reinigung weiter umgesetzt wurde. ¹H-NMR (CDCl₃): δ [ppm] = 7.77 (d, ³J_{HH} = 7.8 Hz, 2H), 7.53 (dd, ³J_{HH} = 7.8 Hz, ³J_{HH} = 7.8 Hz , 2H), 7.34 (d, ³J_{HH} = 7.8 Hz, 2H), 5.78 (br. s, 1H, OH), 1.92 (s, 3H, CH₃).

### b) 1,1-Bis(6-brom-2-pyridyl)-1-fluorethan

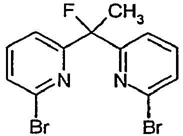

Zu einer auf 10 °C gekühlten Lösung von 105.9 g (296 mmol) 1,1-Bis(6-brom-2-pyridyl)ethan-1-ol in 1500 ml Chloroform wurden während 30 min. 117.3 ml (888 mmol) DAST so zugetropft, daß die Temperatur 20 °C nicht überschritt. Die Reaktionsmischung wurde 1 h bei 20°C gerührt und dann unter Eiskühlung tropfenweise mit 500 ml Eiswasser (Vorsicht: stark exotherme Reaktion) und anschließend mit 1000 ml wäßriger 3 M NaOH hydrolysiert. Die organische Phase wurde abgetrennt, die wäßrige Phase wurde zweimal mit 100 ml Chloroform extrahiert, die vereinigten organischen Phasen wurden einmal mit 500 ml Wasser gewaschen und über Calciumchlorid getrocknet. Nach Abfiltrieren des Trockenmittels wurde die braune organische Phase auf 200 ml eingeengt und über eine Kieselgelsäule filtriert. Die so erhaltene gelbe Lösung wurde bis zur Trockene eingeengt und das verbliebene gelbe, zähe Öl aus 200 ml n-Heptan umkristallisiert, wobei 78.6 g (218 mmol) des Produkts, entsprechend einer Ausbeute von 73.7 %, in Form von farblosen Kristallnadeln - Reinheit nach ¹H-NMR > 99.0 % - erhalten wurde.
¹H-NMR (CDCl₃): δ [ppm] = 7.56 (dd, ³J_{HH} = 7.8 Hz, ³J_{HH} = 7.8 Hz , 2H), 7.50 (d, ³J_{HH} = 7.8 Hz, 2H), 7.34 (d, ³J_{HH} = 7.8 Hz, 2H), 2.15 (d, ³J_{HF} = 23.4 Hz, 3H, CH₃).

### c) 1,1-Bis(6-phenyl-2-pyridyl)-1-fluorethan

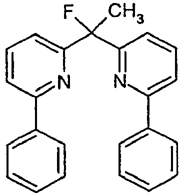

Eine entgaste Suspension von 18.0 g (50 mmol) 1,1-Bis(6-brom-2-pyridyl)-1-fluorethan, 24.4 g (200 mmol) Benzolboronsäure und 19.2 g (330 mmol) Kaliumfluorid in 350 ml THF wurde mit 600 µl (2.6 mmol) Tri-tert-butylphosphin und 449 mg (2.0 mmol) Palladium(II)acetat versetzt und unter Rühren 3 h unter Rückfluß erhitzt. Nach Erkalten wurde das THF im Vakuum entfernt, der Rückstand wurde in 500 ml Dichlormethan aufgenommen und dreimal mit 300 ml Wasser gewaschen. Nach Trocknen über Magnesiumsulfat, Filtration über Kieselgel und Abziehen des Lösungsmittels wurde des verbleibende gelbe Öl dreimal aus Ethanol umkristallisiert, wobei 15.7 g (44 mmol) des Produkts, entsprechend einer Ausbeute von 88.6 %, in Form von farblosen Kristallnadeln - Reinheit nach ¹H-NMR > 99% - erhalten wurde.
¹H-NMR (CDCl₃): δ [ppm] = 8.04 (d, ³J_{HH} = 7.7 Hz, 4H), 7.72 (dd, ³J_{HH} = 7.8 Hz, ³J_{HH} = 7.8 Hz , 2H), 7.63 (d, ³J_{HH} = 7.8 Hz, 2H), 7.50 (d, ³J_{HH} = 7.8 Hz, 2H), 7.44 - 7.35 (m, 6H), 2.35 (d, ³J_{HF} = 23.4 Hz, 3H, CH₃).

### Komplexsynthese

### Beispiel 1: [1,1-Bis(6-phenyl-2-pyridinato-N,C²)-1-fluorethan]platin(II)

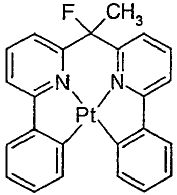

Eine Lösung von 1.063 g (3.0 mmol) 1,1-Bis(6-phenyl-2-pyridyl)-1-fluorethan und 1.144 g (3.0 mmol) *cis*-Dimethyl-di(η¹-S-dimethylsulfoxidyl)platin(II) in 15 ml Toluol wurde 3 h bei 90 °C gerührt. Nach Erkalten auf Raumtemperatur wurde die gelbe Suspension mit 30 ml Diethylether versetzt, das gelbe, mikrokristalline Produkt wurde abgesaugt und dreimal mit je 10 ml Diethylether gewaschen. Nach Trocknen im Vakuum wurden 1.544 g (2.8 mmol), entsprechend einer Ausbeute von 94.0 %, mit einer Reinheit > 99.5% (HPLC), erhalten.
MS (FAB): m/e = 347 (M+).

## Patentansprüche

1. Elektrisch neutrale Verbindungen gemäß Struktur 1, 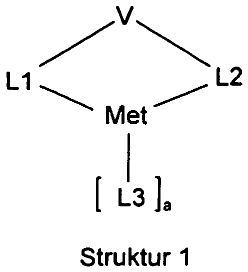 **dadurch gekennzeichnet, daß** sie ein Metall Met enthalten, koordiniert an einen vierzähnig chelatisierenden Liganden Lig gemäß Struktur 2, 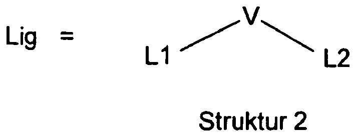 wobei V eine verbrückende Einheit ist, **dadurch gekennzeichnet, daß** sie 1 bis 40 Atome aus der vierten, fünften und/oder sechsten Hauptgruppe enthält und die zwei Teilliganden L1 und L2, die gleich oder verschieden bei jedem Auftreten sein können, kovalent miteinander verbindet, und wobei die zwei Teilliganden L1 und L2 der Struktur 3 genügen, 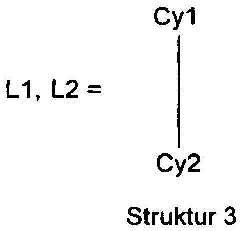 wobei Cy1 und Cy2, gleich oder verschieden bei jedem Auftreten, einem substituierten oder unsubstituierten, gesättigten, ungesättigten oder aromatischen Homo- oder Heterocyclus entsprechen, der jeweils über ein Ringatom oder über ein exocyclisch an den Homo- oder Heterocyclus gebundenes Atom ionisch, kovalent oder koordinativ an das Metall gebunden ist;
und wobei L3 gleich oder verschieden bei jedem Auftreten ein ein- oder zweizähniger, neutraler oder monoanionischer Ligand ist und wobei a gleich 0, 1 oder 2 ist;
dabei sind die folgenden Verbindungen von der Erfindung ausgenommen: 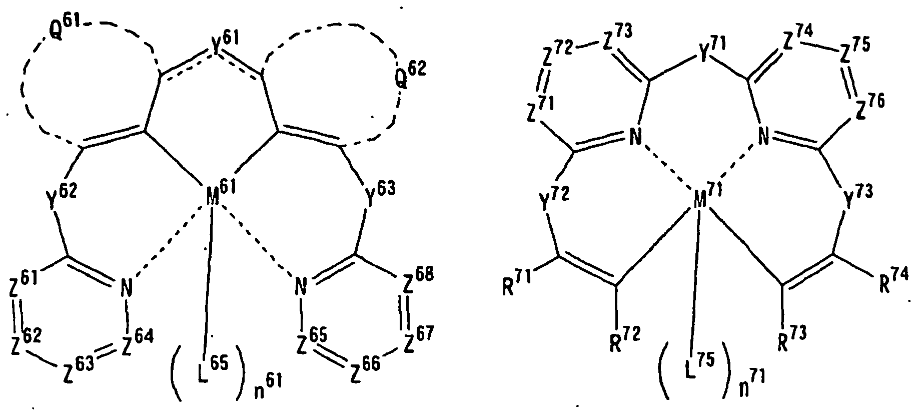 worin gilt:
M⁶¹, M⁷¹ ist ein Metallion;
Y⁶¹, Y⁷¹ ist eine Carbonylgruppe oder eine Alkylengruppe;
Y⁶², Y⁶³, Y⁷², Y⁷³ ist jeweils eine Einfachbindung;
Q⁶¹, Q⁶² stellen eine Gruppe dar, die einen Ring bilden;
Z⁶¹ bis Z⁶⁸, Z⁷¹ bis Z⁷⁶ stellen jeweils ein substituiertes oder unsubstituiertes Kohlenstoffatom oder ein Stickstoffatom dar;
R⁷¹ bis R⁷⁴ ist jeweils ein Substituent, wobei R⁷¹ und R⁷² aneinander binden und so einen Ring bilden und wobei R⁷³ und R⁷⁴ aneinander binden und so einen Ring bilden;
L⁶⁵, L⁷⁵ ist ein Ligand, der an das Metall koordiniert;
n⁶¹, n⁷¹ ist 0 bis 4.

2. Verbindungen gemäß Anspruch 1, **dadurch gekennzeichnet, daß** L1 = L2 ist.

3. Verbindungen gemäß Anspruch 1 oder 2, **dadurch gekennzeichnet, daß** die verbrückende Einheit V 1 bis 6 Atome enthält oder ein 3- bis 6-gliedriger Homo- oder Heterocyclus ist.

4. Verbindungen gemäß einem oder mehreren der Ansprüche 1 bis 3, **dadurch gekennzeichnet, daß** für die Verknüpfungseinheit V gilt:
V ist C=O, C=NR¹, C=S, CR₂, CR(OH), CR(OR¹), C(NR¹)₂, -(CR₂)R₂C(CR₂)-, -(CR₂CR₂)R₂C(CR₂CR₂)-, -(SiR₂)R₂C(SiR₂)-. -(SiR₂CR₂)R₂C(CR₂SiR₂)-, - (CR₂SiR₂)R₂C(SiR₂CR₂)-, -(SiR₂SiR₂)R₂C(SiR₂SiR₂)-, cis-RC=CR, 1,2-C₆H₄, 1,3-C₆H₄, SiR₂, Si(OH)₂, Si(OR¹)₂, -(CR₂)R₂Si(CR₂)-, -(CR₂CR₂)R₂Si(CR₂CR₂)-, -(SiR₂)R₂Si(SiR₂)-, -(SiR₂CR₂)R₂Si(CR₂SiR₂)-, -(CR₂SiR₂)R₂Si(SiR₂CR₂)-, -(SiR₂SiR₂)R₂Si(SiR₂SiR₂)-, R'N, -(CR₂)R¹N(CR₂)-, -(CR₂CR₂)R¹N(CR₂CR₂)-, FP, FPO, R¹P, R¹As, R¹Sb, R¹Si, R¹PO, R¹AsO, R¹SbO, R¹BiO, R¹PSe, R¹AsSe, R¹SbSe, R¹SiSe, R¹PTe, R¹AsTe, R¹SbTe, R'BiTe, -O-R¹PO-O-, -O-(R¹O)PO-O-, -CR₂O-R¹PO-OCR₂-, -OCR₂-R¹PO-CR₂O-, O, S, Se, -(CR₂)O(CR₂)-, -(CR₂)S(CR₂)-, -(CR₂)(O)S(CR₂)- oder -(CR₂)(O)₂S(CR₂)- oder entsprechende unsymmetrische Analoga;
R ist gleich oder verschieden bei jedem Auftreten H, F, Cl, Br, I, NO₂, CN, eine geradkettige, verzweigte oder cyclische Alkyl- oder Alkoxygruppe mit 1 bis 20 C-Atomen, wobei eine oder mehrere nicht benachbarte CH₂-Gruppen durch -R¹C=CR¹-, -C≡C-, Si(R¹)₂, Ge(R¹)₂, Sn(R¹)₂, C=O, C=S, C=Se, C=NR¹, -O-, -S-, -NR¹- oder -CONR¹- ersetzt sein können und wobei ein oder mehrere H-Atome durch F ersetzt sein können, oder eine Aryl-, Aryloxy- oder Heteroarylgruppe mit 1 bis 14 C-Atomen, die durch einen oder mehrere nicht aromatische Reste R substituiert sein kann, wobei mehrere Substituenten R wiederum ein weiteres mono- oder polycyclisches, aliphatisches oder aromatisches Ringsystem aufspannen können; und
R¹, R² ist gleich oder verschieden bei jedem Auftreten H oder ein aliphatischer oder aromatischer Kohlenwasserstoffrest mit 1 bis 20 C-Atomen.

5. Metallkomplexe gemäß einem oder mehreren der Ansprüche 1 bis 4, ausgewählt aus den Verbindungen (1) bis (8), die jeweils noch einen oder zwei zusätzliche Liganden L3 tragen können, 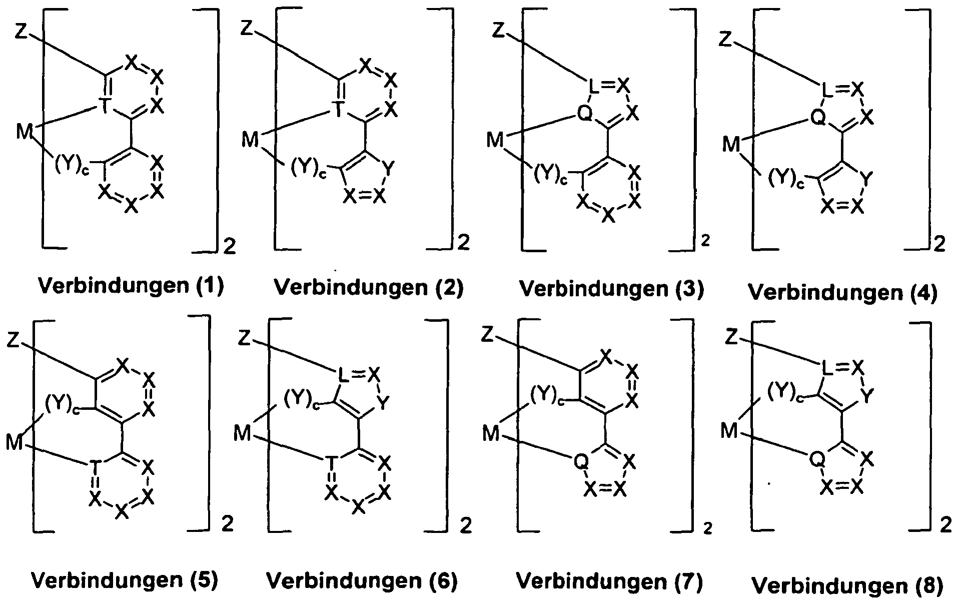 wobei R, R¹ und R² dieselbe Bedeutung haben, wie in Anspruch 4 beschrieben, und die weiteren Symbole und Indizes folgende Bedeutung haben:
M Be, Mg, Ca, Sr, Ba, Al, Ga, In, TI, Sc, Y, La, Cr, Mo, W, Fe, Ru, Os, Co, Rh, Ir, Ni, Pd, Pt, Cu, Ag, Au, Zn, Cd, Hg;
L ist gleich oder verschieden bei jedem Auftreten C, N, P;
Q ist gleich oder verschieden bei jedem Auftreten N, O, S, Se, Te;
T ist gleich oder verschieden bei jedem Auftreten N, P;
X ist gleich oder verschieden bei jedem Auftreten CR, N, P;
Y ist gleich oder verschieden bei jedem Auftreten NR¹, O, S, Se, Te, SO, SeO, TeO, SO₂, SeO₂, TeO₂;
Z hat dieselbe Bedeutung, wie in Anspruch 4 für V beschrieben;
c ist gleich oder verschieden bei jedem Auftreten 0 oder 1.

6. Metallkomplexe gemäß einem oder mehreren der Ansprüche 1 bis 4, ausgewählt aus den Verbindungen (9) bis (12), die jeweils noch einen oder zwei zusätzliche Liganden L3 tragen können, 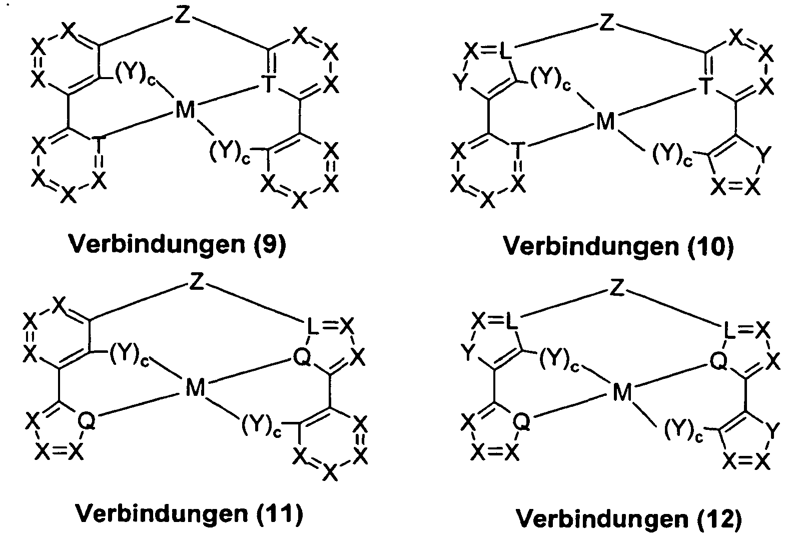 wobei die Symbole und Indizes M, L, Q, T, X, Y, Z, R, R¹, R² und c die gleichen Bedeutungen wie in Anspruch 4 und 5 haben.

7. Metallkomplexe gemäß einem oder mehreren der Ansprüche 1 bis 4, ausgewählt aus den Verbindungen (13) bis (30), die jeweils noch einen oder zwei zusätzliche Liganden L3 tragen können, 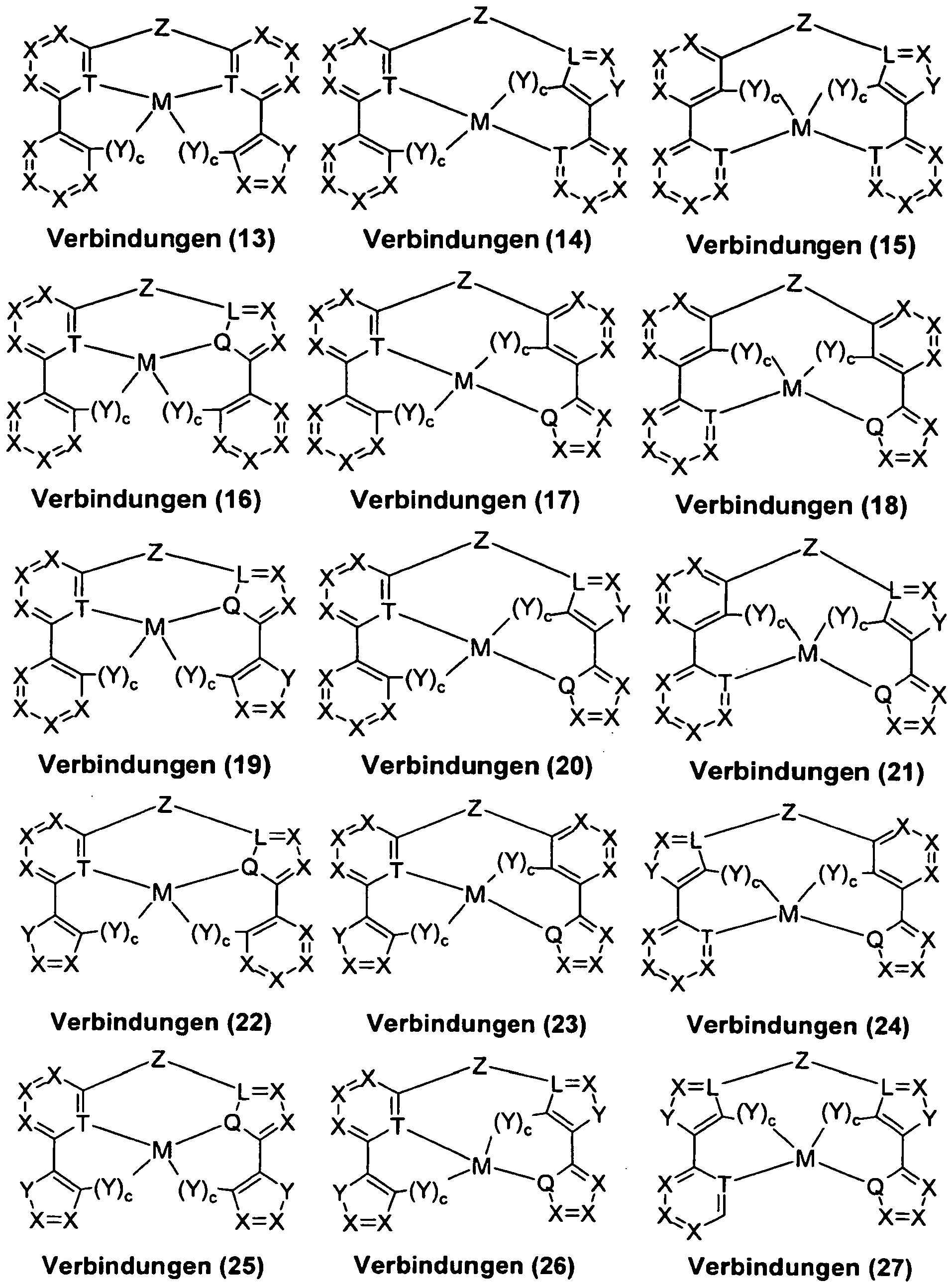 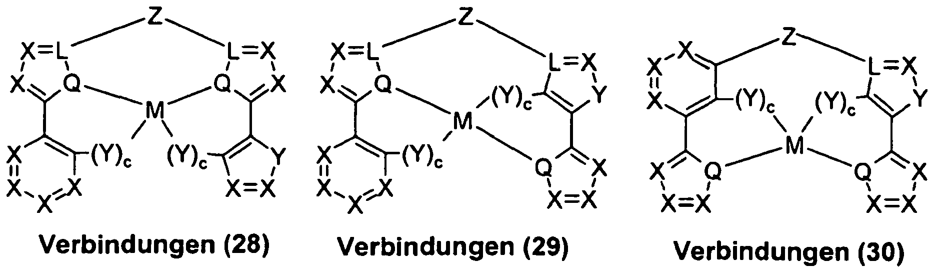 wobei die Symbole und Indizes M, L, Q, T, X, Y, Z, R, R¹, R², c und n die Bedeutungen wie in Anspruch 4 und 5 haben.

8. Metallkomplexe gemäß einem oder mehreren der Ansprüche 1 bis 7, **dadurch gekennzeichnet, daß** der Ligand L3, wenn vorhanden, ein zweizähnig chelatisierender Ligand ist.

9. Metallkomplexe gemäß Anspruch 8, **dadurch gekennzeichnet, daß** L3 ein monoanionischer Ligand gleich oder verschieden den Teilliganden L1 bzw. L2 ist oder daß L3 ein Ligand gemäß Struktur (4) ist, 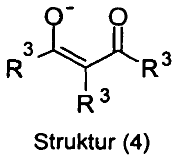 wobei R³ gleich oder verschieden bei jedem Auftreten H, eine C₁ bis C₂₀ Alkylgruppe, eine C₁ bis C₂₀ Alkoxygruppe, eine C₄ bis C₂₀ Aryl- oder Heteroarylgruppe oder eine C₄ bis C₂₀ Aryloxy- oder Heteroaryloxygruppe darstellt und ein oder mehrere H-Atome durch F ersetzt sein können.

10. Verbindungen gemäß einem oder mehreren der Ansprüche 5 bis 9, **dadurch gekennzeichnet, daß** das Symbol M = Be, Mg, Pt oder Zn bedeutet und der Index a = 0 ist.

11. Verbindungen gemäß Anspruch 10, **dadurch gekennzeichnet, daß** das Symbol c = 0 und M = Pt bedeutet.

12. Verbindungen gemäß einem oder mehreren der Ansprüche 5 bis 9, **dadurch gekennzeichnet, daß** für das Symbol M = Rh oder Ir gilt und der Index a = 1 mit einem zweizähnigen monoanionischen Liganden L3 oder a = 2 mit einzähnigen monoanionischen Liganden L3 ist.

13. Verbindungen gemäß einem oder mehreren der Ansprüche 5 bis 12, **dadurch gekennzeichnet, daß** das Symbol L = C oder N bedeutet.

14. Verbindungen gemäß einem oder mehreren der Ansprüche 5 bis 13, **dadurch gekennzeichnet, daß** das Symbol Q = O oder S bedeutet.

15. Verbindungen gemäß einem oder mehreren der Ansprüche 5 bis 14, **dadurch gekennzeichnet, daß** das Symbol T = N bedeutet.

16. Verbindungen gemäß einem oder mehreren der Ansprüche 5 bis 15, **dadurch gekennzeichnet, daß** das Symbol X = CR oder N bedeutet.

17. Verbindungen gemäß einem oder mehreren der Ansprüche 5 bis 16, **dadurch gekennzeichnet, daß** das Symbol Z = CR₂, CO, SiR¹₂, R¹N, FP, FPO, R¹P, R¹PO, - CR₂CR₂-, -CR₂-O-CR₂-, -O-(OR¹)PO-O-, cis-CR=CR, -CR₂-CO-CR₂-, -CR₂-CR₂-CR₂- oder -CR₂-NR¹-CR₂ bedeutet.

18. Verbindungen gemäß einem oder mehreren der Ansprüche 5 bis 17, **dadurch gekennzeichnet, daß** das Symbol R = H, F, Cl, Br, I, CN, eine geradkettige, verzweigte oder cyclische Alkyl- oder Alkoxygruppe mit 1 bis 6 C-Atomen oder eine Aryl- oder Heteroarylgruppe mit 3 bis 10 C-Atomen, die durch einen oder mehrere, nicht aromatische Reste R substituiert sein kann, wobei mehrere Substituenten R, sowohl am selben Ring als auch an den beiden unterschiedlichen Ringen zusammen wiederum ein weiteres mono- oder polycyclisches Ringsystem aufspannen können, ist.

19. Verfahren zur Herstellung der Verbindungen gemäß einem oder mehreren der Ansprüche 1 bis 18, durch Umsetzung der Verbindungen (31) bis (60) 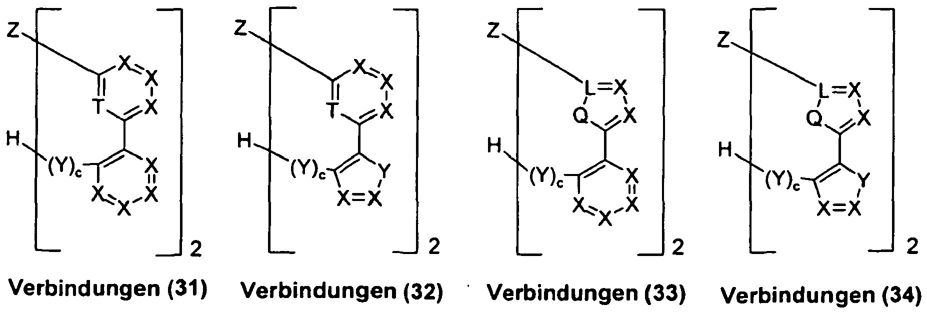 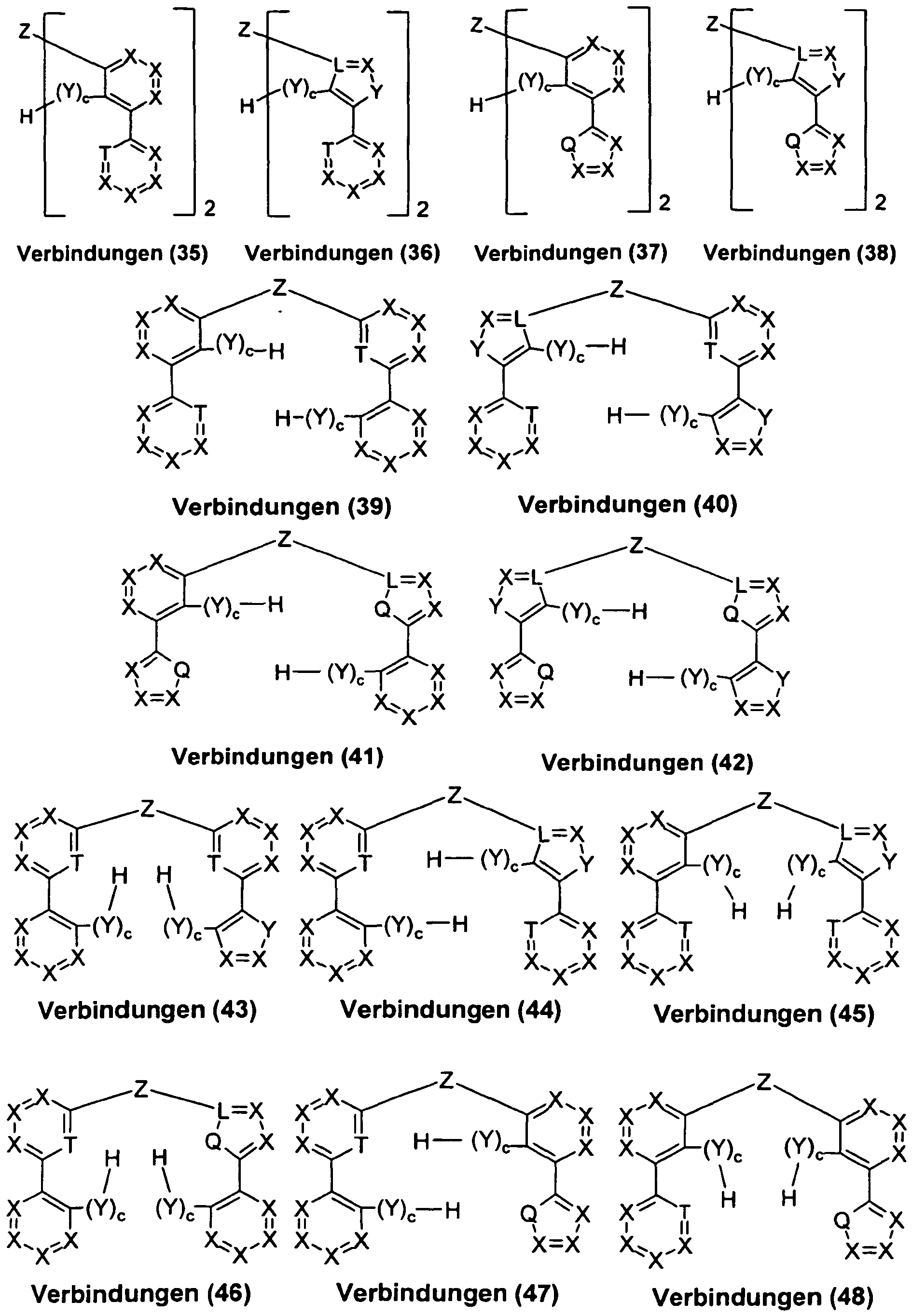 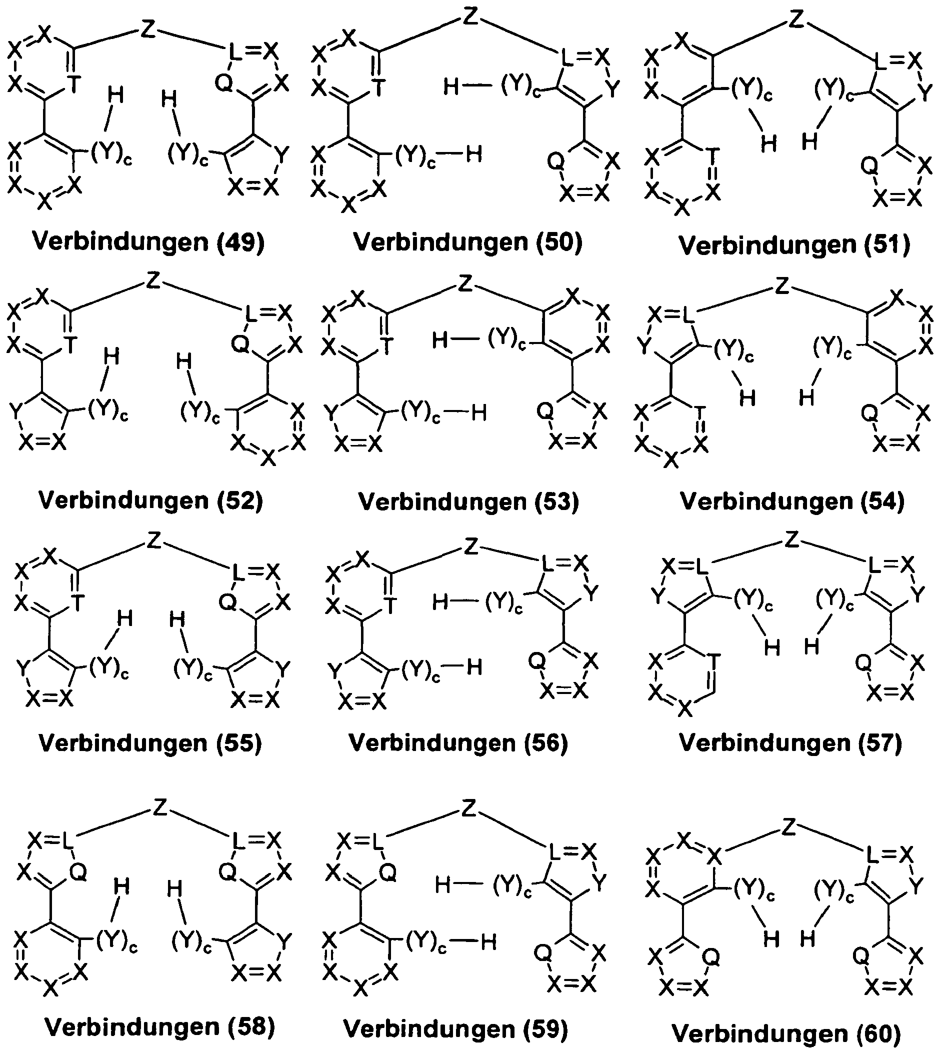 wobei die Symbole und Indizes L, Q, T, X, Y, Z, R, R¹, R² und c die gleichen Bedeutungen wie in Anspruch 4 und 5 haben, ausgenommen die Verbindungen Bis(6-phenyl-2-pyridyl)methan, Bis(6-phenyl-2-pyridyl)keton, Bis(6-(1-hydroxy-3,5-di-tert-butyl)phenyl-2-pyridyl)methanol, 2,2'-Thio-bis(3-cyano-2,4-diphenyl]pyridin, Bis(6-(3-phenyl)phenyl-2-pyridyl)methan,
mit Metallalkoholaten der Verbindung (61), mit Metallketoketonaten der Verbindung (62), Metallhalogeniden -carboxylaten, -nitraten, -sulfaten der Verbindung (63) oder Metallalkyl- bzw. -arylverbindungen der Verbindung (64), 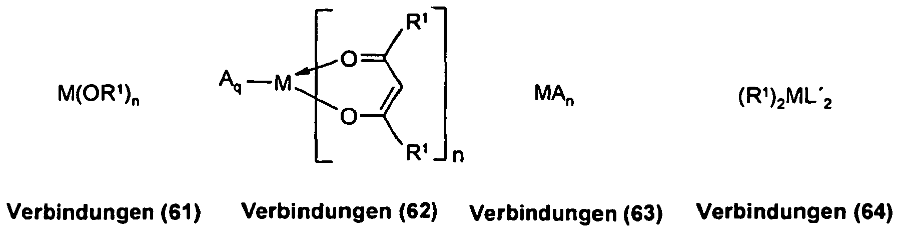 wobei die Symbole M und R¹ die in Anspruch 5 und 6 genannte Bedeutung haben, und das Symbol A = F, Cl, Br, I, OH, Formiat, Acetat, Propionat, Benzoat, Nitrat oder Sulfat und L' ein einzähniger Ligand und n = 1, 2 oder 3 und q = 0, 1, 2 oder 3 ist.

20. Verbindungen gemäß einem oder mehreren der Ansprüche 1 bis 18, **dadurch gekennzeichnet, daß** ihre Reinheit (mittels ¹H-NMR und/oder HPLC bestimmt) mehr als 99 % beträgt.

21. Konjugierte, teilkonjugierte und/oder nicht-konjugierte Polymere oder Dendrimere enthaltend eine oder mehrere Verbindungen der Struktur 1 bzw. Verbindungen (1) bis (30) gemäß einem oder mehreren der Ansprüche 1 bis 18.

22. Polymere oder Dendrimere gemäß Anspruch 21, **dadurch gekennzeichnet, daß** mindestens ein in Anspruch 4 definierter Rest R eine Bindung zum Polymer oder Dendrimer darstellt.

23. Polymere gemäß Anspruch 21 und/oder 22, **dadurch gekennzeichnet, daß** das Polymer aus der Gruppe Polyfluorene, Poly-spirobifluorene, Poly-para-phenylene, Poly-dihydrophenanthrene, Poly-indenofluorene, Poly-carbazole, Polythiophene, Polyketone, Polyvinylcarbazole oder auch aus Copolymeren, die mehrere der hier genannten Einheiten aufweisen, ausgewählt ist.

24. Elektronisches Bauteil, enthaltend mindestens eine Verbindung, ein Polymer, ein Copolymer oder ein Dendrimer gemäß einem oder mehreren der Ansprüche 1 bis 18 und 20 bis 23.

25. Elektronisches Bauteil gemäß Anspruch 24, **dadurch gekennzeichnet, daß** es sich um eine Organische Leuchtdiode (OLEDs), eine Organische Integrierte Schaltung (O-ICs), einen Organischen Feld-Effekt-Transistor (OFETs), einen Organischen Dünnfilmtransistor (OTFTs), eine Organische Solarzelle (O-SCs) oder eine Organische Laserdiode (O-Laser) handelt.

## Claims

1. Electrically neutral compounds of structure 1, 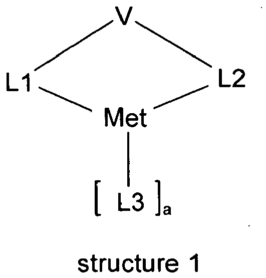 **characterised in that** they contain a metal Met, coordinated to a tetradentate chelating ligand Lig of structure 2, 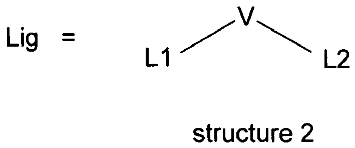 where V is a bridging unit, **characterised in that** it contains 1 to 40 atoms from the fourth, fifth and/or sixth main group and connects the two ligand moieties L1 and L2, which may be identical or different on each occurrence, covalently to one another, and where the two ligand moieties L1 and L2 satisfy structure 3, 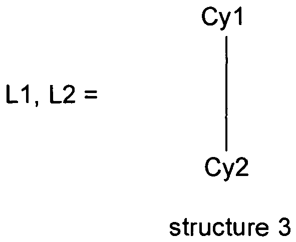 where Cy1 and Cy2, identically or differently on each occurrence, correspond to a substituted or unsubstituted, saturated, unsaturated or aromatic homo- or heterocyclic ring, which is in each case ionically, covalently or coordinatively bonded to the metal via a ring atom or via an atom which is exocyclically bonded to the homo- or heterocyclic ring;
and where L3, identically or differently on each occurrence, is a mono- or bidentate, neutral or monoanionic ligand, and where a is equal to 0, 1 or 2;
the following compounds are excluded from the invention: 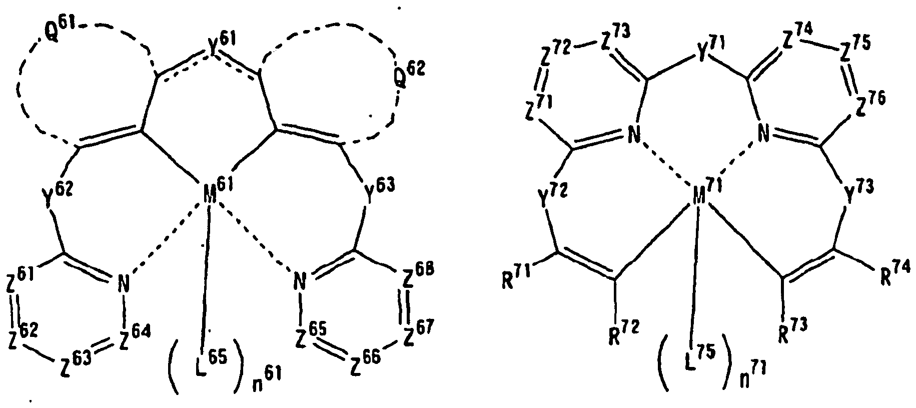 in which:
M⁶¹, M⁷¹ are a metal ion;
Y⁶¹, Y⁷¹ are a carbonyl group or an alkylene group;
Y⁶², Y⁶³, Y⁷², Y⁷³ are each a single bond;
Q⁶¹, Q⁶² represents a group which forms a ring;
Z⁶¹ to Z⁶⁸, Z⁷¹ to Z⁷⁶ each represent a substituted or unsubstituted carbon atom or a nitrogen atom;
R⁷¹ to R⁷⁴ are each a substituent, where R⁷¹ and R⁷² are bonded to one another and thus form a ring and where R⁷³ and R⁷⁴ are bonded to one another and thus form a ring;
L⁶⁵ , L⁷⁵ are a ligand which is coordinated to the metal;
n⁶¹, n⁷¹ are 0 to 4.

2. Compounds according to Claim 1, **characterised in that** L1 = L2.

3. Compounds according to Claim 1 or 2, **characterised in that** the bridging unit V contains 1 to 6 atoms or is a 3- to 6-membered homo- or heterocyclic ring.

4. Compounds according to one or more of Claims 1 to 3, **characterised in that** the following applies to the linking unit V:
V is C=O, C==NR¹, C=S, CR₂, CR(OH), CR(OR¹), C(NR¹)₂, -(CR₂)R₂C(CR₂)-, -(CR₂CR₂)R₂C(CR₂CR₂)-, -(SiR₂)R₂C(SiR₂)-, -(SiR₂CR₂)R₂C(CR₂SiR₂)-, -(CR₂SiR₂)R₂C(SiR₂CR₂)-, -(SiR₂SiR₂)R₂C(SiR₂SiR₂)-, cis-RC=CR, 1,2-C₆H₄, 1,3-C₆H₄, SiR₂, Si(OH)₂, Si(OR¹)₂, -(CR₂)R₂Si(CR₂)-, -(CR₂CR₂)R₂Si(CR₂CR₂)-, -(SiR₂)R₂Si(SiR₂)-, -(SiR₂CR₂)R₂Si(CR₂SiR₂)-, -(CR₂SiR₂)R₂Si(SiR₂CR₂)-, -(SiR₂SiR₂)R₂Si(SiR₂SiR₂)-, R¹N, -(CR₂)R¹N(CR₂)-, -(CR₂CR₂)R¹N(CR₂CR₂)-, FP, FPO, R¹P, R¹As, R¹Sb, R¹Bi, R¹PO, R¹AsO, R¹SbO, R¹BiO, R¹PSe, R¹AsSe, R¹SbSe, R¹BiSe, R¹PTe, R¹AsTe, R¹SbTe, R¹BiTe, -O-R¹PO-O-, -O-(R¹O)PO-O-, -CR₂OR¹PO-OCR₂-, -OCR₂-R¹PO-CR₂O-, O, S, Se, -(CR₂)O(CR₂)-, -(CR₂)S(CR₂)-, -(CR₂)(O)S(CR₂)- or -(CR₂)(O)₂S(CR₂)- or corresponding asymmetrical analogues;
R is, identically or differently on each occurrence, H, F, Cl, Br, I, NO₂, CN, a straight-chain, branched or cyclic alkyl or alkoxy group having 1 to 20 C atoms, where one or more non-adjacent CH₂ groups may be replaced by -R¹C=CR¹- -C≡C-, Si(R¹)₂ Ge(R¹)₂, Sn(R¹)₂, C=O, C=S, C=Se, C=NR¹, -O-, -S-, -NR¹- or -CONR¹- and where one or more H atoms may be replaced by F, or an aryl, aryloxy or heteroaryl group having 1 to 14 C atoms, which may be substituted by one or more non-aromatic radicals R, where a plurality of substituents R may in turn form a further mono- or polycyclic, aliphatic or aromatic ring system; and
R¹, R² are, identically or differently on each occurrence, H or an aliphatic or aromatic hydrocarbon radical having 1 to 20 C atoms.

5. Metal complexes according to one or more of Claims 1 to 4, selected from compounds (1) to (8), each of which may also carry one or two additional ligands L3, 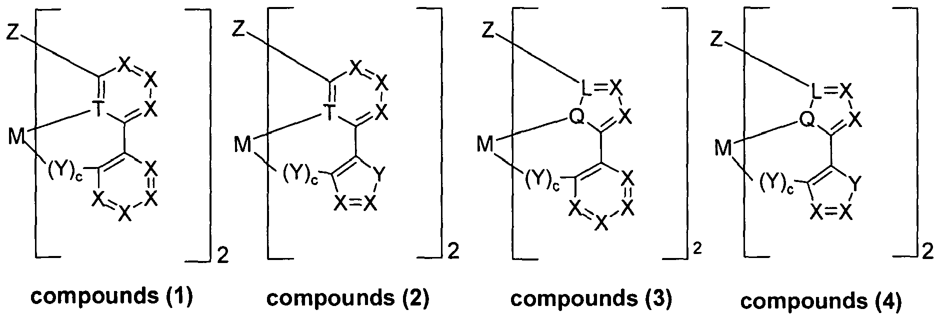 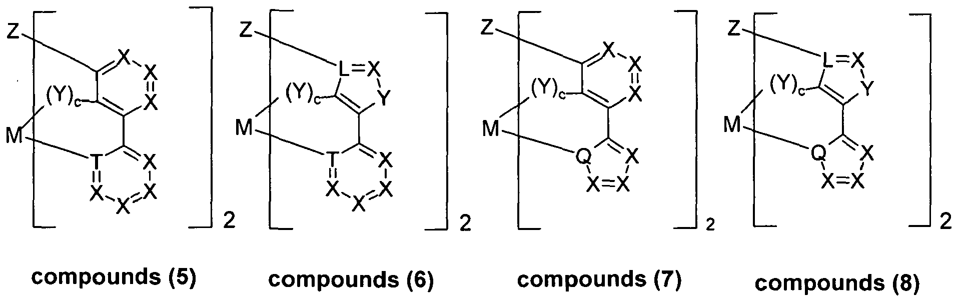 where R, R¹ and R² have the same meaning as described in Claim 4, and the other symbols and indices have the following meaning:
M is Be, Mg, Ca, Sr, Ba, Al, Ga, In, TI, Sc, Y, La, Cr, Mo, W, Fe, Ru, Os, Co, Rh, Ir, Ni, Pd, Pt, Cu, Ag, Au, Zn, Cd, Hg;
L is, identically or differently on each occurrence, C, N, P;
Q is, identically or differently on each occurrence, N, O, S, Se, Te;
T is, identically or differently on each occurrence, N, P;
X is, identically or differently on each occurrence, CR, N, P;
Y is, identically or differently on each occurrence, NR¹, O, S, Se, Te, SO, SeO, TeO, SO₂, SeO₂, TeO₂;
Z has the same meaning as described for V in Claim 4;
c is, identically or differently on each occurrence, 0 or 1.

6. Metal complexes according to one or more of Claims 1 to 4, selected from compounds (9) to (12), each of which may also carry one or two additional ligands L3, 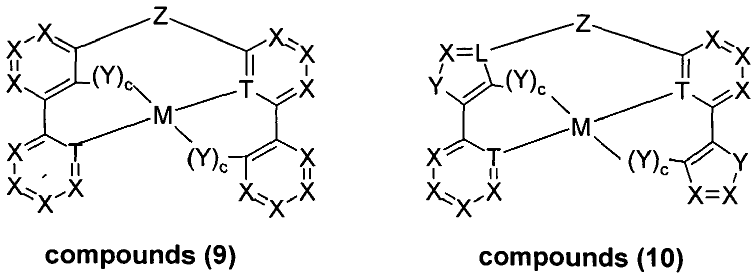 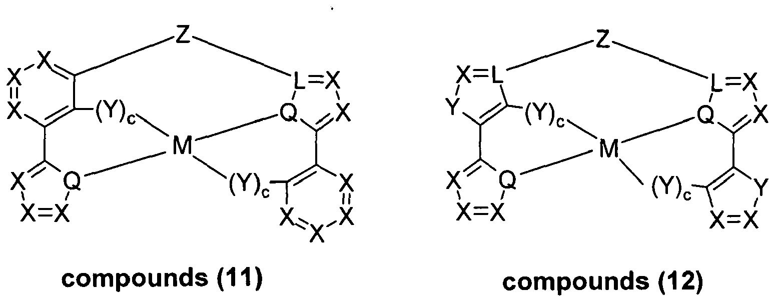 where the symbols and indices M, L, Q, T, X, Y, Z, R, R¹, R² and c have the same meanings as in Claims 4 and 5.

7. Metal complexes according to one or more of Claims 1 to 4, selected from compounds (13) to (30), each of which may also carry one or two additional ligands L3, 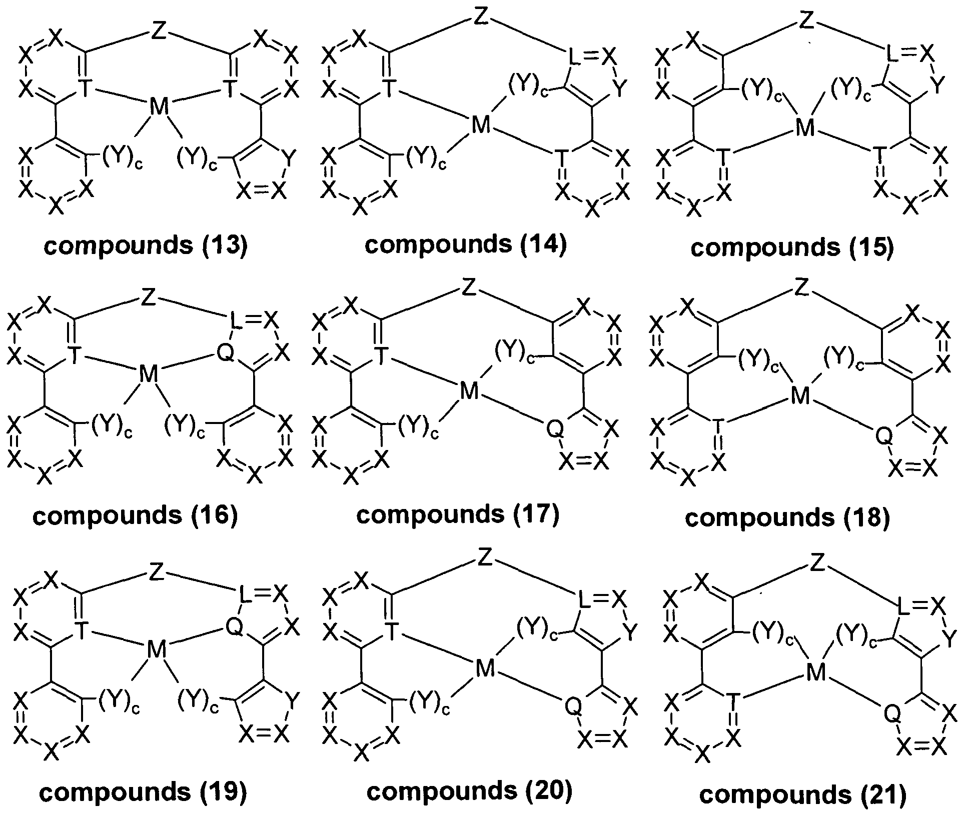 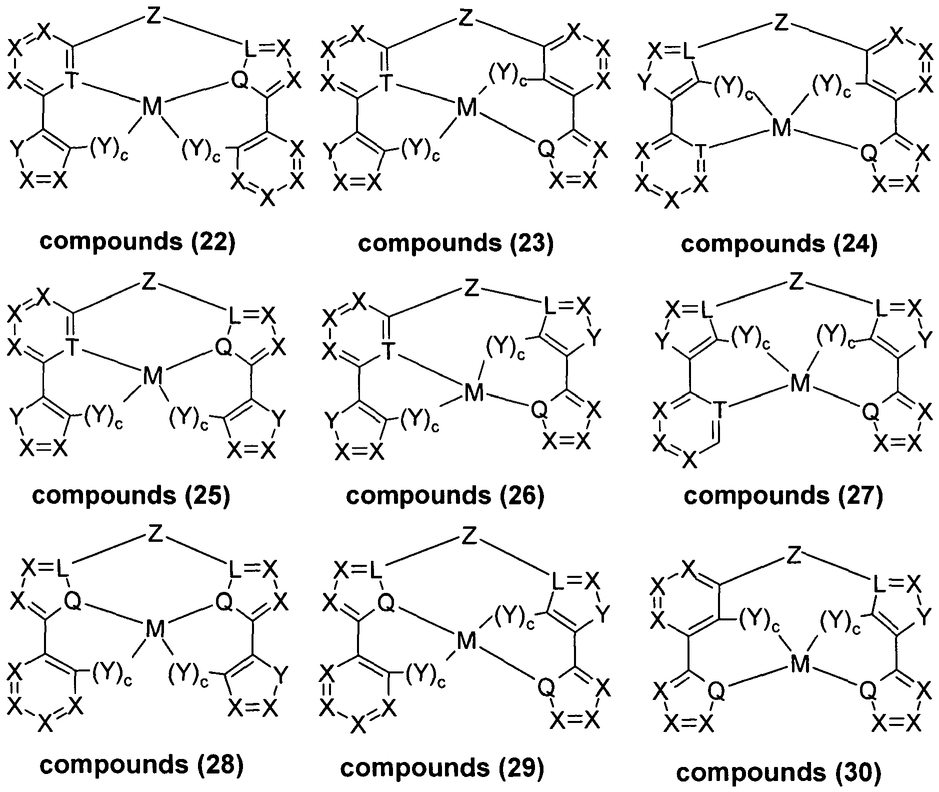 where the symbols and indices M, L, Q, T, X, Y, Z, R, R¹, R², c and n have the meanings as in Claims 4 and 5.

8. Metal complexes according to one or more of Claims 1 to 7, **characterised in that** ligand L3, if present, is a bidentate chelating ligand.

9. Metal complexes according to Claim 8, **characterised in that** L3 is a monoanionic ligand which is identical to or different from ligand moieties L1 and L2 or **in that** L3 is a ligand of structure (4), 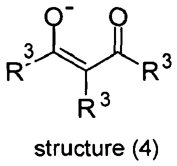 where R³, identically or differently on each occurrence, represents H, a C₁ to C₂₀ alkyl group, a C₁ to C₂₀ alkoxy group, a C₄ to C₂₀ aryl or heteroaryl group or a C₄ to C₂₀ aryloxy or heteroaryloxy group, and one or more H atoms may be replaced by F.

10. Compounds according to one or more of Claims 5 to 9, **characterised in that** the symbol M = Be, Mg, Pt or Zn, and the index a = 0.

11. Compounds according to Claim 10, **characterised in that** the symbols c = 0 and M = Pt.

12. Compounds according to one or more of Claims 5 to 9, **characterised in that** the symbol M = Rh or Ir, and the index a = 1 in the case of a bidentate monoanionic ligand L3 or a = 2 in the case of monodentate monoanionic ligands L3.

13. Compounds according to one or more of Claims 5 to 12, **characterised in that** the symbol L = C or N.

14. Compounds according to one or more of Claims 5 to 13, **characterised in that** the symbol Q = O or S.

15. Compounds according to one or more of Claims 5 to 14, **characterised in that** the symbol T = N.

16. Compounds according to one or more of Claims 5 to 15, **characterised in that** the symbol X = CR or N.

17. Compounds according to one or more of Claims 5 to 16, **characterised in that** the symbol Z = CR₂, CO, SiR¹₂, R¹N, FP, FPO, R¹P, R¹PO, -CR₂CR₂-, -CR₂-O-CR₂-, -O-(OR¹)PO-O-, cis-CR=CR, -CR₂-CO-CR₂-, -CR₂-CR₂-CR₂- or -CR₂-NR¹-CR₂.

18. Compounds according to one or more of Claims 5 to 17, **characterised in that** the symbol R = H, F, Cl, Br, I, CN, a straight-chain, branched or cyclic alkyl or alkoxy group having 1 to 6 C atoms or an aryl or heteroaryl group having 3 to 10 C atoms, which may be substituted by one or more non-aromatic radicals R, where a plurality of substituents R, both on the same ring and also on the two different rings, may together in turn form a further mono- or polycyclic ring system.

19. Process for the preparation of the compounds according to one or more of Claims 1 to 18, by reaction of compounds (31) to (60) 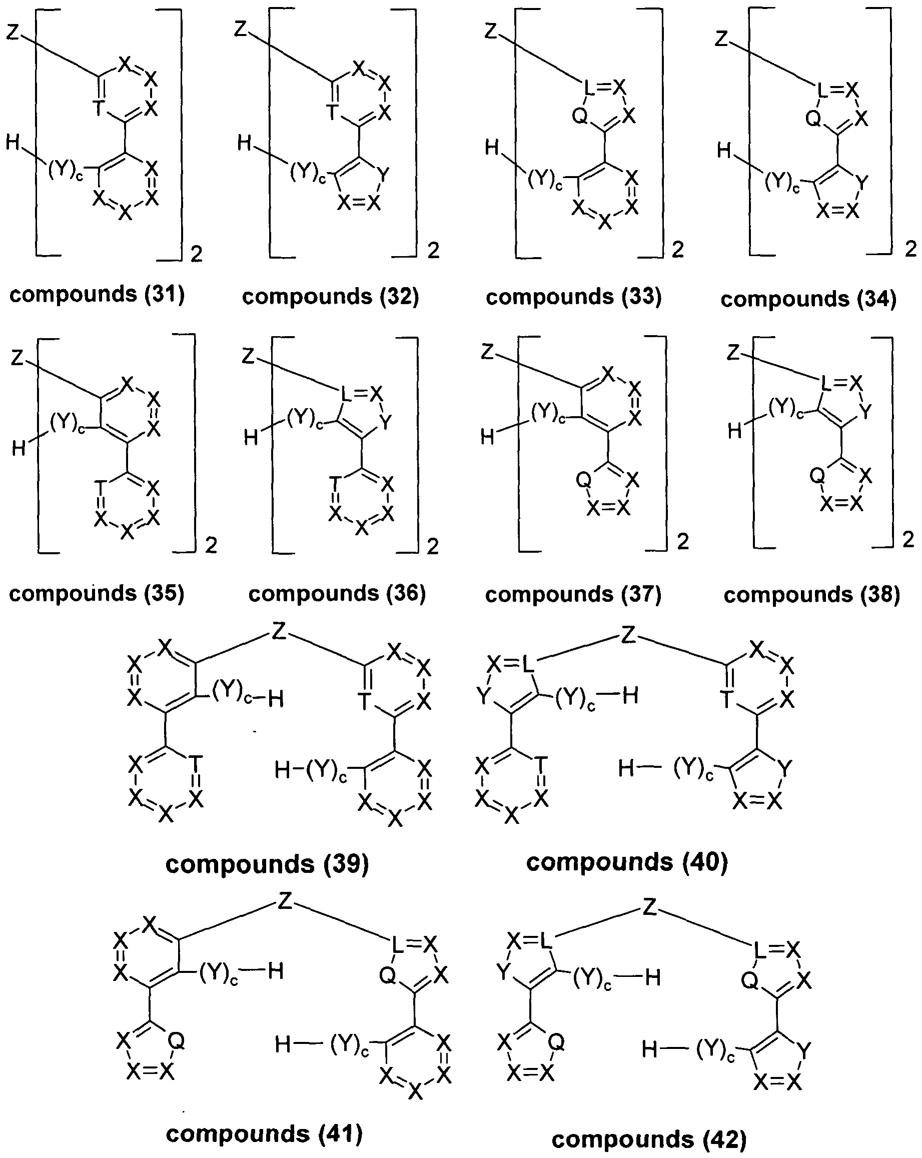 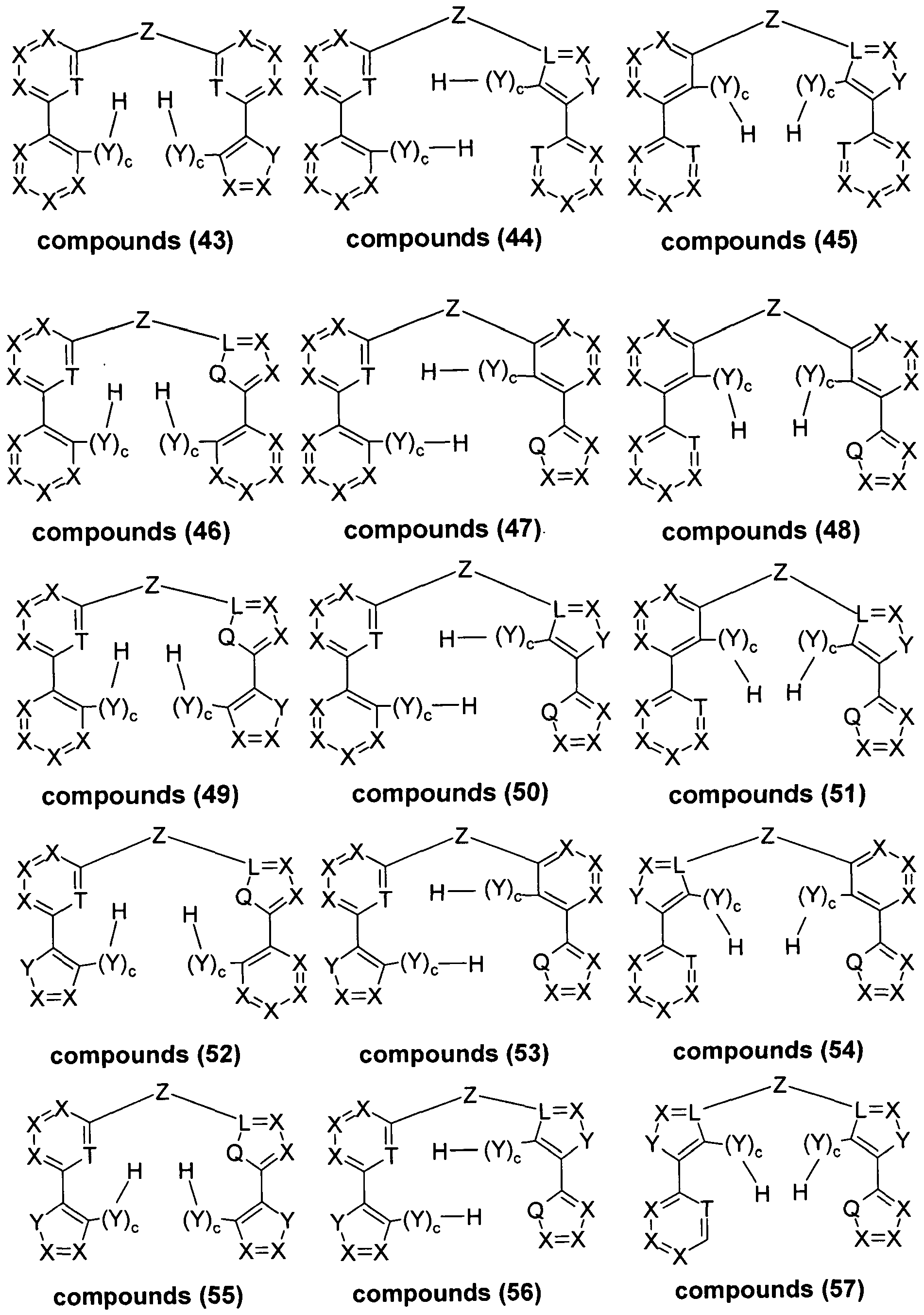 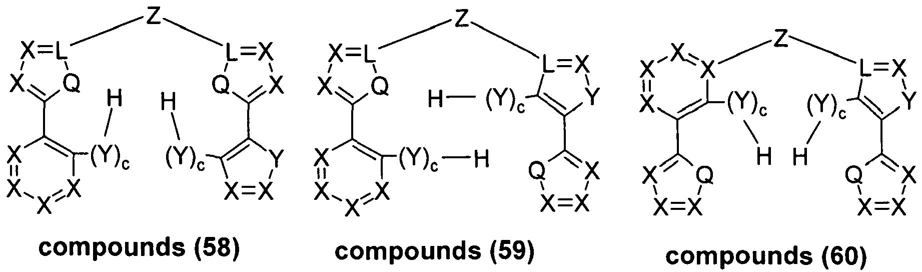 where the symbols and indices L, Q, T, X, Y, Z, R, R¹, R² and c have the same meanings as in Claims 4 and 5, with the exception of the compounds bis(6-phenyl-2-pyridyl)methane, bis(6-phenyl-2-pyridyl) ketone, bis(6-(1-hydroxy-3,5-di-tert-butyl)-phenyl-2-pyridyl)methanol, 2,2'-thiobis(3-cyano-2,4-diphenyl)pyridine, bis(6-(3-phenyl)phenyl-2-pyridyl)methane,
with metal alcoholates of compound (61), with metal ketoketonates of compound (62), metal halides, carboxylates, nitrates and sulfates of compound (63) or alkyl- or arylmetal compounds of compound (64), 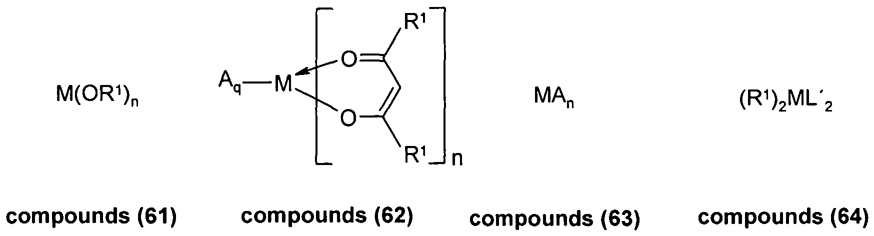 where the symbols M and R¹ have the meaning given in Claims 5 and 6, and the symbol A = F, Cl, Br, I, OH, formate, acetate, propionate, benzoate, nitrate or sulfate, and L' is a monodentate ligand, and n = 1, 2 or 3, and q = 0, 1, 2 or 3.

20. Compounds according to one or more of Claims 1 to 18, **characterised in that** their purity (determined by means of ¹H-NMR and/or HPLC) is greater than 99%.

21. Conjugated, partially conjugated and/or non-conjugated polymers or dendrimers containing one or more compounds of structure 1 or compounds (1) to (30) according to one or more of Claims 1 to 18.

22. Polymers or dendrimers according to Claim 21, **characterised in that** at least one radical R defined in Claim 4 represents a bond to the polymer or dendrimer.

23. Polymers according to Claim 21 and/or 22, **characterised in that** the polymer is selected from the group polyfluorenes, polyspirobifluorenes, poly-para-phenylenes, polydihydrophenanthrenes, polyindenofluorenes, polycarbazoles, polythiophenes, polyketones, polyvinylcarbazoles or from copolymers which have a plurality of the units mentioned here.

24. Electronic component comprising at least one compound, a polymer, a copolymer or a dendrimer according to one or more of Claims 1 to 18 and 20 to 23.

25. Electronic component according to Claim 24, **characterised in that** it is an organic light-emitting diode (OLED), an organic integrated circuit (O-IC), an organic field-effect transistor (OFET), an organic thin-film transistor (OTFT), an organic solar cell (O-SC) or an organic laser diode (O-laser).

## Revendications

1. Composés électriquement neutres de la structure 1, 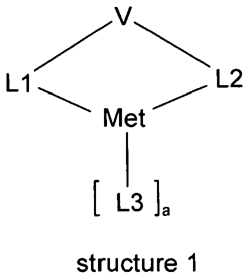 **caractérisés en ce qu'**ils contiennent un métal Met, coordonné à un ligand chélatant tétradenté Lig de la structure 2, 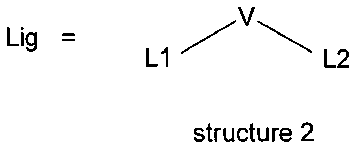 où V est une unité de pontage, **caractérisée en ce qu'**elle contient de 1 à 40 atomes à partir du quatrième, cinquième et/ou sixième groupe principal et **en ce qu'**elle connecte les deux moitiés de ligand L1 et L2, qui peuvent être identiques ou différentes à chaque occurrence, de façon covalente l'une à l'autre, et où les deux moitiés de ligand L1 et L2 satisfont la structure 3, 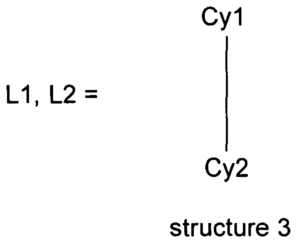 où Cy1 et Cy2, de façon identique ou différente à chaque occurrence, correspondent à un cycle homo- ou hétérocyclique saturé, non saturé ou aromatique, substitué ou non substitué, qui est dans chaque cas de façon ionique, de façon covalente ou de façon coordonnée lié à un métal via un atome de cycle ou via un atome qui est de façon exocyclique lié au cycle homo- ou hétérocyclique ;
et où L3, de façon identique ou différente à chaque occurrence, est un ligand neutre ou monoanionique, mono- ou bidenté, et où a est égal à 0, 1 ou 2 ;
les composés suivants étant exclus de l'invention : 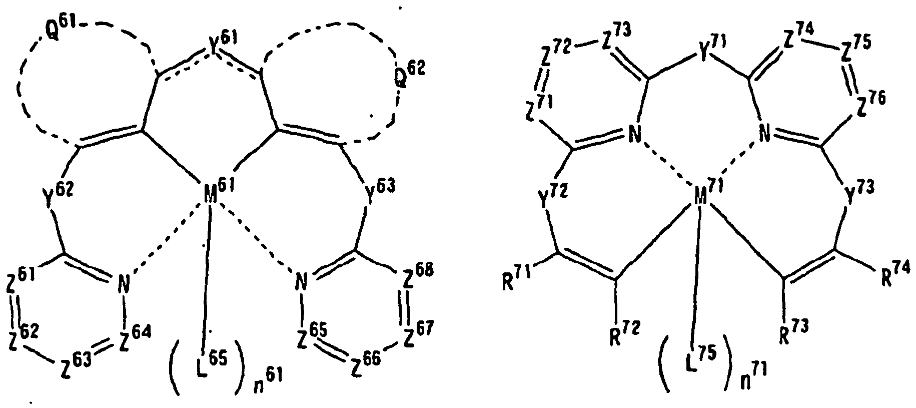 dans lesquels :
M⁶¹ M⁷¹ sont un ion métal ;
Y⁶¹, Y⁷¹ sont un groupe carbonyle ou un groupe alkylène ;
Y⁶², Y⁶³, Y⁷², Y⁷³ sont chacun une liaison simple ;
Q⁶¹, Q⁶² représente un groupe qui forme un cycle ;
Z⁶¹ à Z⁶⁸, Z⁷¹ à Z⁷⁶ représentent chacun un atome de carbone substitué ou non substitué ou un atome d'azote ;
R⁷¹ à R⁷⁴ sont chacun un substituant, où R⁷¹ et R⁷² sont liés l'un à l'autre et forment ainsi un cycle et où R⁷³ et R⁷⁴ sont liés l'un à l'autre et forment ainsi un cycle ;
L⁶⁵, L⁷⁵ sont un ligand qui est coordonné au métal ;
n⁶¹, n⁷¹ sont 0 à 4.

2. Composés selon la revendication 1, **caractérisés en ce que** L1 = L2.

3. Composés selon la revendication 1 ou 2, **caractérisés en ce que** l'unité de pontage V contient de 1 à 6 atomes ou est un cycle homo- ou hétérocyclique à 3 à 6 éléments.

4. Composés selon une ou plusieurs des revendications 1 à 3, **caractérisés en ce que** ce qui suit s'applique à l'unité de liaison V :
V est C=O, C=NR¹, C=S, CR₂, CR(OH), CR(OR¹), C(NR¹)₂, -(CR₂)R₂C(CR₂)-, -(CR₂CR₂)R₂C(CR₂CR₂)-, -(SiR₂)R₂C(SiR₂)-, -(SiR₂CR₂)R₂C(CR₂SiR₂)-, -(CR₂SiR₂)R₂C(SiR₂CR₂)-, -(SiR₂SiR₂)R₂C(SiR₂SiR₂)-, cis-RC=CR, 1,2-C₆H₄, 1,3-C₆H₄, SiR₂, Si(OH)₂, Si(OR¹)₂, -(CR₂)R₂Si(CR₂)-, -(CR₂CR₂)R₂Si(CR₂CR₂)-, -(SiR₂) R₂Si(SiR₂)-, -(SiR₂CR₂)R₂Si(CR₂SiR₂)-, -(CR₂SiR₂)R₂Si(SiR₂CR₂)-, -(SiR₂SiR₂)R₂Si(SiR₂SiR₂)-, R¹N, -(CR₂)R¹N(CR₂)-, -(CR₂CR₂)R¹N(CR₂CR₂)-, FP, FPO, R¹P, R¹As, R¹Sb, R¹Bi, R¹PO, R¹AsO, R¹SbO, R¹BiO, R¹PSe, R¹AsSe, R¹SbSe, R¹BiSe, R¹PTe, R¹AsTe, R¹SbTe, R¹BiTe, -O-R¹PO-O-, -O-(R¹O)PO-O-, -CR₂OR¹PO-OCR₂-, -OCR₂-R¹PO-CR₂O-, O, S, Se, -(CR₂)O(CR₂)-, -(CR₂)S(CR₂)-, -(CR₂)(O)S(CR₂)- ou -(CR₂)(O)₂S(CR₂)- ou des analogues asymétriques correspondants ;
R est, de façon identique ou différente à chaque occurrence, H, F, Cl, Br, I, NO₂, CN, un groupe alkyle ou alcoxy en chaîne droite, ramifié ou cyclique comprenant de 1 à 20 atomes de C, où un ou plusieurs groupes CH₂ non adjacents peuvent être remplacés par -R¹C=CR¹-, -C≡C-, Si(R¹)₂, Ge(R¹)₂, Sn(R¹)₂ C=O, C=S, C=Se, C=NR¹ -O-, -S-, -NR¹- ou -CONR¹- et où un ou plusieurs atomes de H peuvent être remplacés par F, ou un groupe aryle, aryloxy ou hétéroaryle comprenant de 1 à 14 atomes de C, qui peuvent être substitués par un ou plusieurs radicaux non aromatiques R, où une pluralité de substituants R peuvent à leur tour former un autre système de cycle aliphatique ou aromatique, mono- ou polycyclique ; et
R¹, R² sont, de façon identique ou différente à chaque occurrence, H ou un radical hydrocarbone aliphatique ou aromatique comprenant de 1 à 20 atomes de C.

5. Complexes métalliques selon une ou plusieurs des revendications 1 à 4, choisis parmi les composés (1) à (8), dont chacun peut également porter un ou deux ligands additionnels L3, 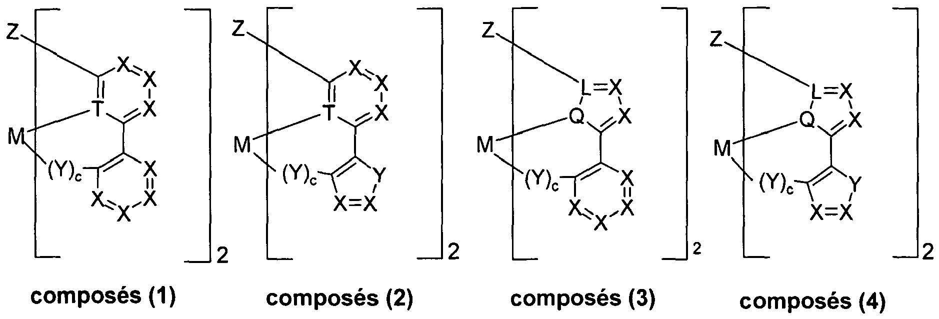 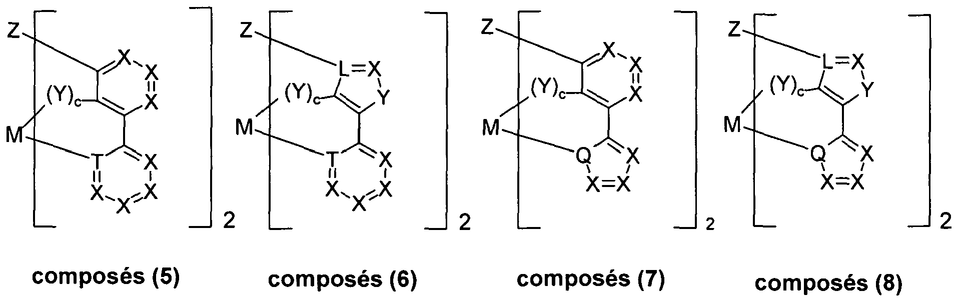 où R, R¹ et R² ont les mêmes significations que celles décrites dans la revendication 4, et les autres symboles et indices ont les significations qui suivent :
M est Be, Mg, Ca, Sr, Ba, Al, Ga, In, Tl, Sc, Y, La, Cr, Mo, W, Fe, Ru, Os, Co, Rh, Ir, Ni, Pd, Pt, Cu, Ag, Au, Zn, Cd, Hg ;
L est, de façon identique ou différente à chaque occurrence, C, N, P ;
Q est, de façon identique ou différente à chaque occurrence, N, O, S, Se, Te;
T est, de façon identique ou différente à chaque occurrence, N, P ;
X est, de façon identique ou différente à chaque occurrence, CR, N, P ;
Y est, de façon identique ou différente à chaque occurrence, NR¹, O, S, Se, Te, SO, SeO, TeO, SO₂, SeO₂, TeO₂ ;
Z a la même signification que celle décrite pour V dans la revendication 4 ;
c est, de façon identique ou différente à chaque occurrence, 0 ou 1.

6. Complexes métalliques selon une ou plusieurs des revendications 1 à 4, choisis parmi les composés (9) à (12), dont chacun peut également porter un ou deux ligands additionnels L3, 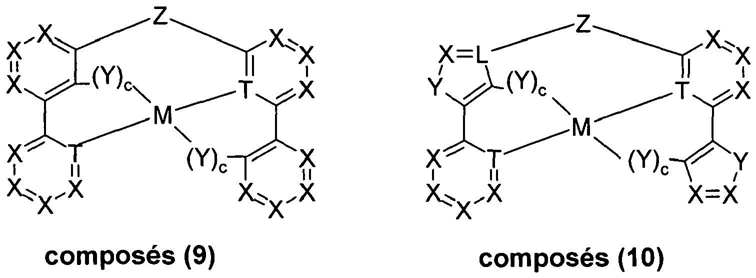 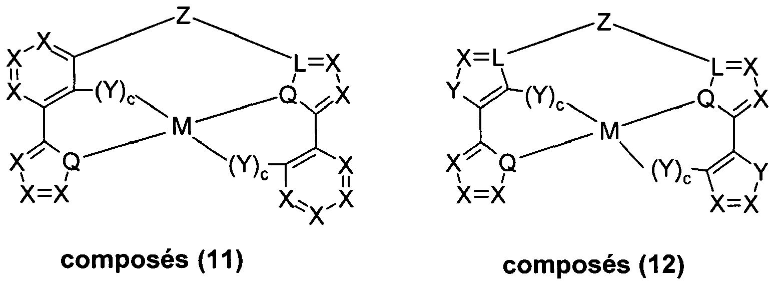 où les symboles et indices M, L, Q, T, X, Y, Z, R, R¹, R² et c ont les mêmes significations que celles dans les revendications 4 et 5.

7. Complexes métalliques selon une ou plusieurs des revendications 1 à 4, choisis parmi les composés (13) à (30), dont chacun peut également porter un ou deux ligands additionnels L3, 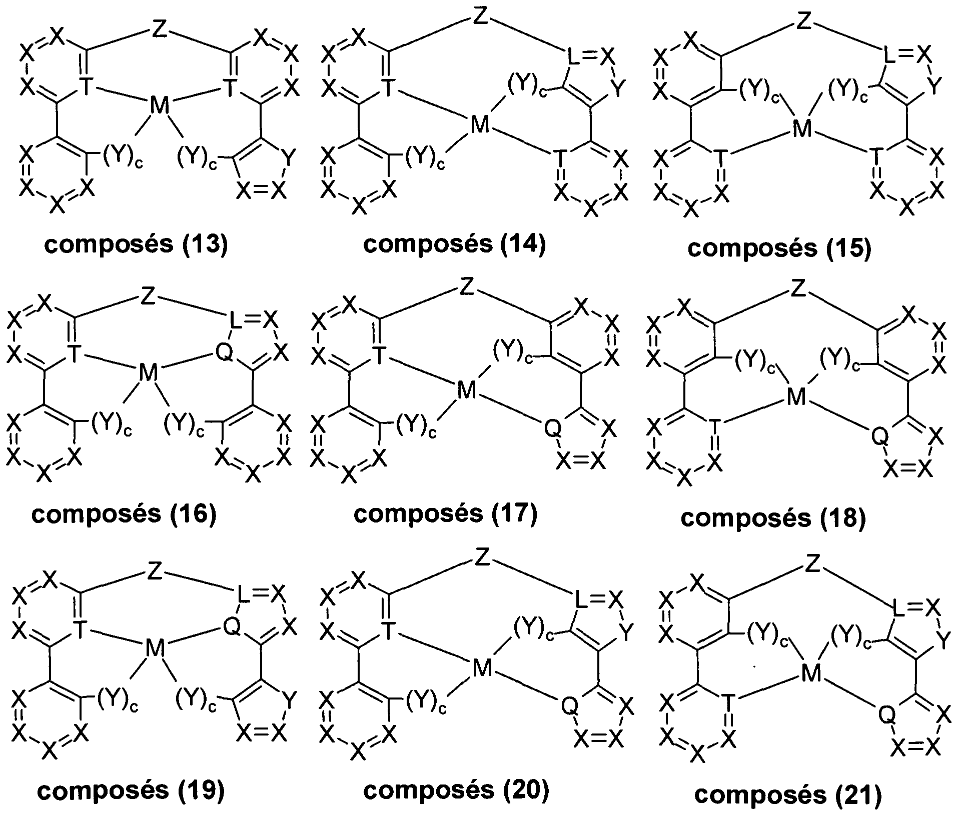 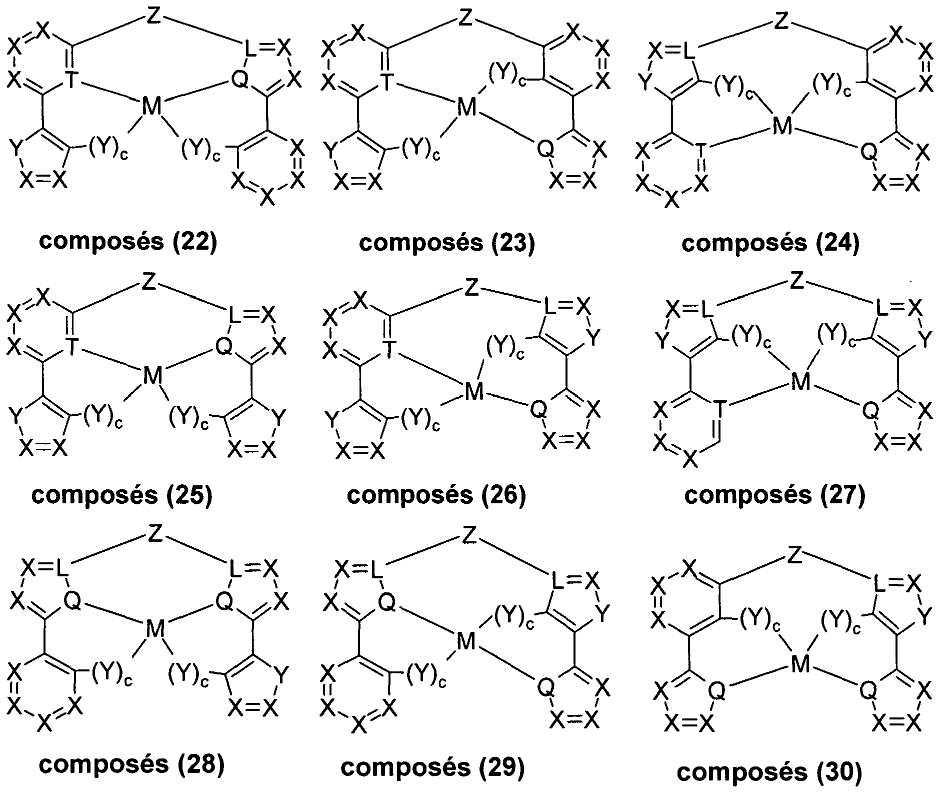 où les symboles et indices M, L, Q, T, X, Y, Z, R, R¹, R², c et n ont les mêmes significations que celles dans les revendications 4 et 5.

8. Complexes métalliques selon une ou plusieurs des revendications 1 à 7, **caractérisés en ce que** le ligand L3, s'il est présent, est un ligand chélatant bidenté.

9. Complexes métalliques selon la revendication 8, **caractérisés en ce que** L3 est un ligand monoanionique qui est identique ou différent des moitiés de ligand L1 et L2 ou **en ce que** L3 est un ligand de la structure (4), 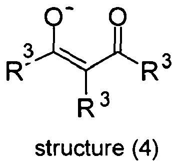 où R³, de façon identique ou différente à chaque occurrence, représente H, un groupe C₁ à C₂₀ alkyle, un groupe C₁ à C₂₀ alcoxy, un groupe C₄ à C₂₀ aryle ou hétéroaryle ou un groupe C₄ à C₂₀ aryloxy ou hétéroaryloxy, et un ou plusieurs atomes de H peuvent être remplacés par F.

10. Composés selon une ou plusieurs des revendications 5 à 9, **caractérisés en ce que** le symbole M = Be, Mg, Pt ou Zn, et l'indice a = 0.

11. Composés selon la revendication 10, **caractérisés en ce que** les symboles c = 0 et M = Pt.

12. Composés selon une ou plusieurs des revendications 5 à 9, **caractérisés en ce que** le symbole M = Rh ou Ir, et l'indice a = 1 dans le cas d'un ligand monoanionique bidenté L3 ou a = 2 dans le cas des ligands monoanioniques monodentés L3.

13. Composés selon une ou plusieurs des revendications 5 à 12, **caractérisés en ce que** le symbole L = C ou N.

14. Composés selon une ou plusieurs des revendications 5 à 13, **caractérisés en ce que** le symbole Q = O ou S.

15. Composés selon une ou plusieurs des revendications 5 à 14, **caractérisés en ce que** le symbole T = N.

16. Composés selon une ou plusieurs des revendications 5 à 15, **caractérisés en ce que** le symbole X = CR ou N.

17. Composés selon une ou plusieurs des revendications 5 à 16, **caractérisés en ce que** le symbole Z = CR₂, CO, SiR¹₂, R¹N, FP, FPO, R¹P, R¹PO, -CR₂CR₂-, -CR₂-O-CR₂-, -O-(OR¹)PO-O-, cis-CR=CR, -CR₂-CO-CR₂-, -CR₂-CR₂-CR₂- ou -CR₂-NR¹-CR₂.

18. Composés selon une ou plusieurs des revendications 5 à 17, **caractérisés en ce que** le symbole R = H, F, Cl, Br, I, CN, un groupe alkyle ou alcoxy en chaîne droite, ramifié ou cyclique comprenant de 1 à 6 atomes de C ou un groupe aryle ou hétéroaryle comprenant de 3 à 10 atomes de C, qui peuvent être substitués par un ou plusieurs radicaux non aromatiques R, où une pluralité de substituants R, à la fois sur le même cycle et également sur les deux cycles différents, peuvent ensemble à leur tour former un autre système de cycle mono- ou polycyclique.

19. Procédé pour la préparation des composés selon une ou plusieurs des revendications 1 à 18, par réaction des composés (31) à (60) 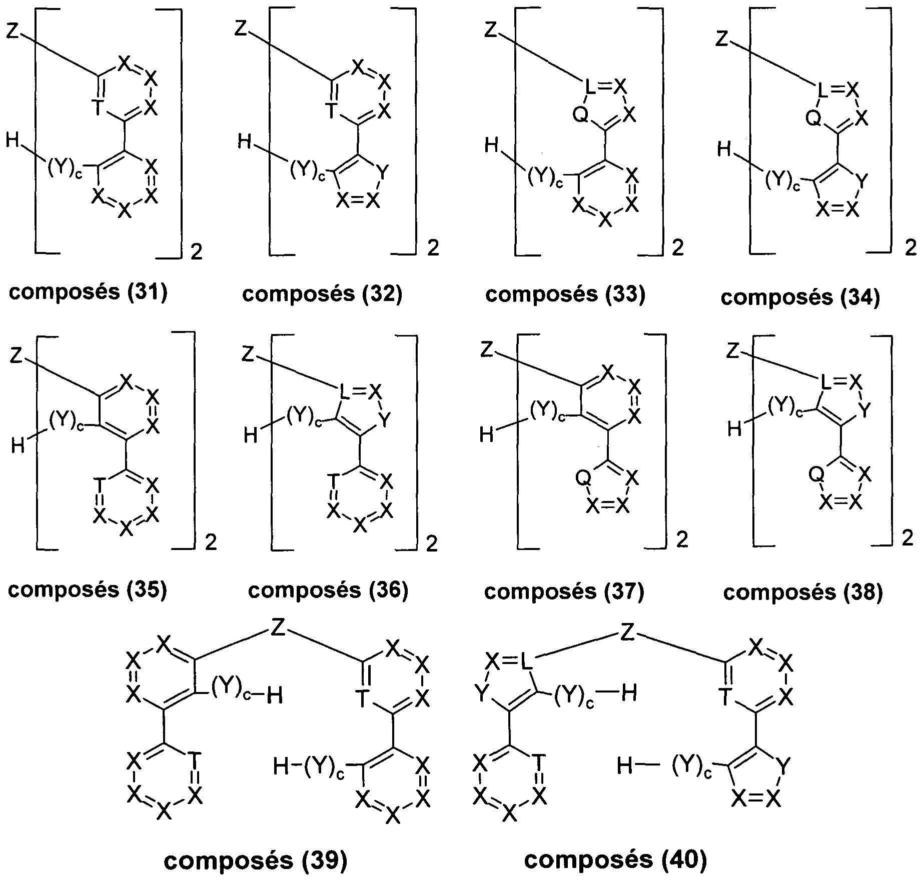 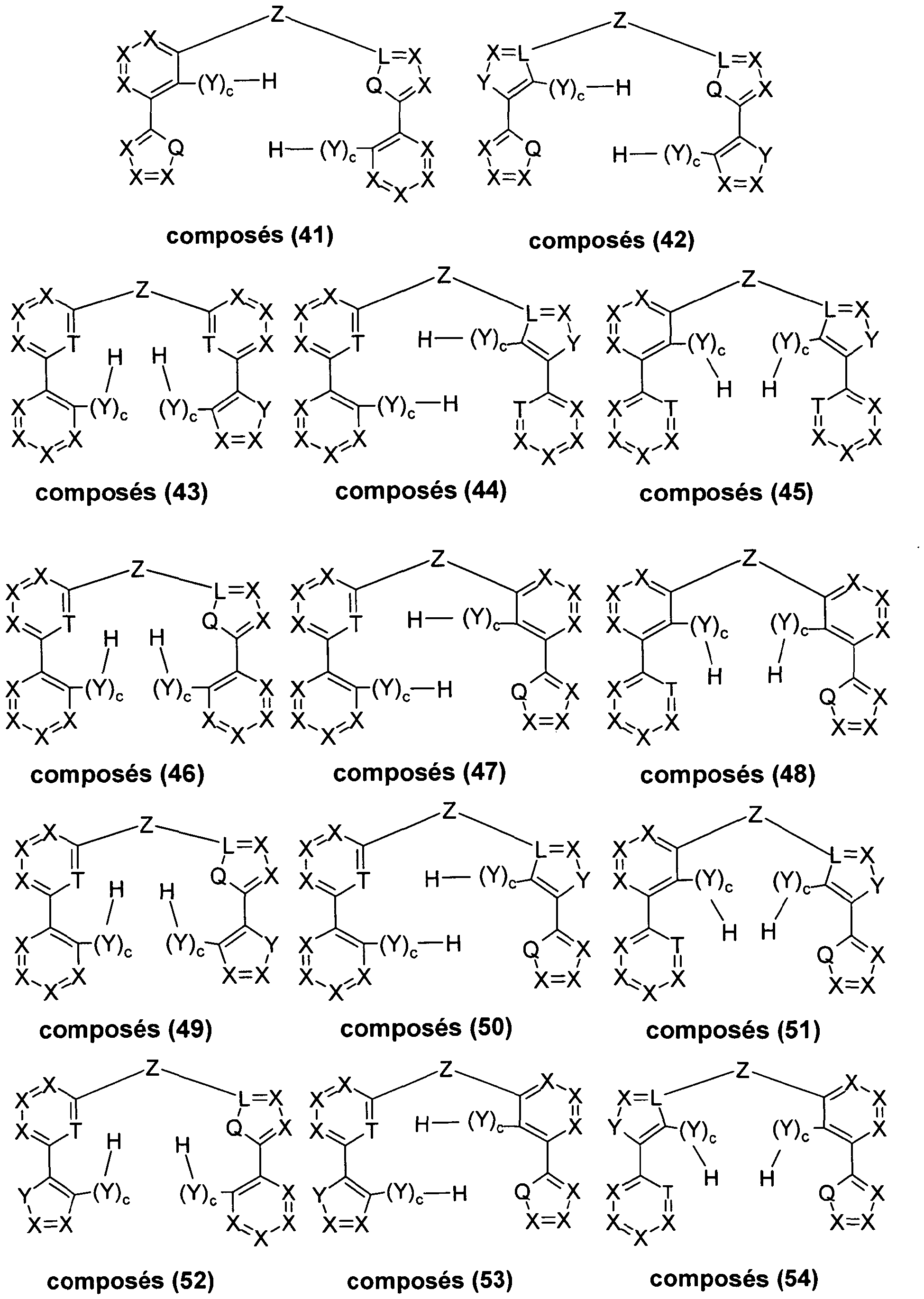 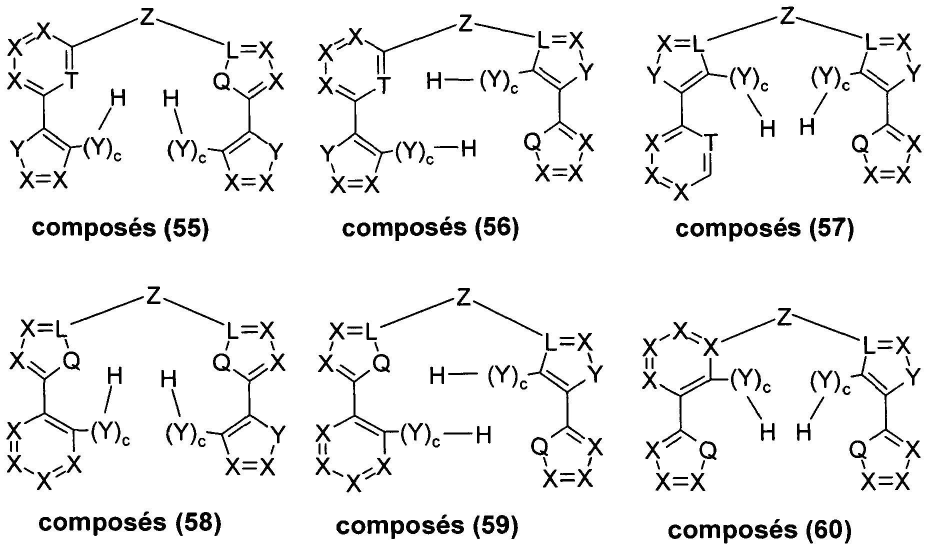 où les symboles et indices L, Q, T, X, Y, Z, R, R¹, R² et c ont les mêmes significations que celles dans les revendications 4 et 5, à l'exception des composés bis(6-phényl-2-pyridyl)méthane, bis(6-phényl-2-pyridyl)cétone, bis(6-(1 -hydroxy-3,5-di-tert-butyl)phényl-2-pyridyl)méthanol, 2,2'-thiobis(3-cyano-2,4-diphényl)pyridine, bis(6-(3-phényl)phényl-2-pyridyl)méthane,
avec des alcoolates de métal du composé (61), avec des cétocétonates de métal du composé (62), des halogénures de métal, des carboxylates, des nitrates et des sulfates du composé (63) ou des composés d'alkyl- ou arylmétal du composé (64), 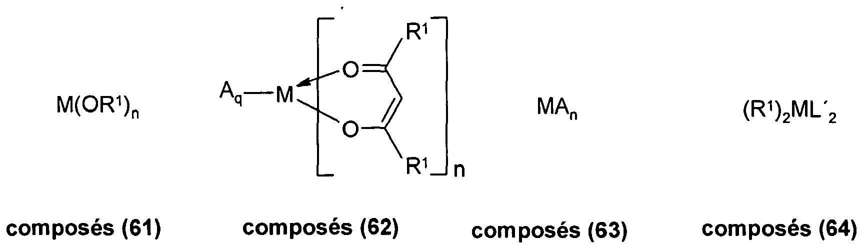 où les symboles M et R¹ ont les significations données dans les revendications 5 et 6, et le symbole A = F, Cl, Br, I, OH, formate, acétate, propionate, benzoate, nitrate ou sulfate, et L' est un ligand monodenté, et n = 1, 2 ou 3, et q = 0, 1, 2 ou 3.

20. Composés selon une ou plusieurs des revendications 1 à 18, **caractérisés en ce que** leur pureté (déterminée au moyen de ¹H-NMR et/ou HPLC) est supérieure à 99%.

21. Polymères ou dendrimères conjugués, partiellement conjugués et/ou non conjugués contenant un ou plusieurs composés de la structure 1 ou composés (1) à (30) selon une ou plusieurs des revendications 1 à 18.

22. Polymères ou dendrimères selon la revendication 21, **caractérisés en ce qu'**au moins un radical R défini dans la revendication 4 représente une liaison avec le polymère ou dendrimère.

23. Polymères selon la revendication 21 et/ou 22, **caractérisés en ce que** le polymère est choisi parmi le groupe polyfluorènes, polyspirobifluorènes, poly-paraphénylènes, polydihydrophénanthrènes, polyindénofluorènes, polycarbazoles, polythiophènes, polycétones, polyvinylcarbazoles ou parmi des copolymères qui comprennent une pluralité des unités mentionnées ici.

24. Composant électronique comprenant au moins un composé, un polymère, un copolymère ou un dendrimère selon une ou plusieurs des revendications 1 à 18 et 20 à 23.

25. Composant électronique selon la revendication 24, **caractérisé en ce qu'**il s'agit d'une diode émettrice de lumière organique (OLED), d'un circuit intégré organique (O-IC), d'un transistor à effet de champ organique (OFET), d'un transistor à film mince organique (OTFT), d'une cellule solaire organique (O-SC) ou d'une diode laser organique (0-laser).
